# EUROPÄISCHE PATENTSCHRIFT

(11) **EP 2 642 960 B1**
(45) Veröffentlichungstag und Bekanntmachung des Hinweises auf die Patenterteilung: **23.03.2016**
(21) Anmeldenummer: 11785607.0
(22) Anmeldetag: 19.11.2011
(51) Int. Cl.: A61F 13/00, A61M 1/00, A61M 27/00

(54) **WUNDAUFLAGE FÜR DIE UNTERDRUCKTHERAPIE**
WOUND DRESSING FOR NEGATIVE PRESSURE THERAPY
PANSEMENT POUR THÉRAPIE À PRESSION NÉGATIVE

(30) Priorität: 25.11.2010 DE 102010052336
(43) Veröffentlichungstag der Anmeldung: 02.10.2013
(73) Patentinhaber: Paul Hartmann AG, 89522 Heidenheim (DE)
(72) Erfinder: CROIZAT, Pierre, 89542 Herbrechtingen (DE); ECKSTEIN, Axel, 89522 Heidenheim (DE); FINK, Ulrich, 89522 Heidenheim (DE); HOFSTETTER, Jürgen, 89522 Heidenheim (DE)
(86) Internationale Anmeldenummer: PCT/EP2011/005840
(87) Internationale Veröffentlichungsnummer: WO 2012/069167

(56) Entgegenhaltungen:
- WO-A1-01/85248
- WO-A1-03/057307
- WO-A1-2010/072309
- WO-A1-2011/076340
- US-A1- 2005 070 858

## Beschreibung

Die vorliegende Erfindung betrifft einen Verband zur Verwendung bei der Unterdrucktherapie von Wunden, insbesondere von Wunden im Abdominalbereich, umfassend eine flexible Folie als Wundkontaktschicht und mindestens ein auf die Folie aufgebrachtes Leitungsmittel, wobei das Leitungsmittel durch Öffnungen in dem Leitungsmittel und in der Folie mit dem Wundraum Fluid-leitend verbindbar ist.

Vorrichtungen zur Unterdrucktherapie von Wunden sind im Stand der Technik bekannt. So beschreibt beispielsweise die WO1993/00972 eine Vorrichtung zur Förderung der Wundheilung durch die Applikation von Unterdruck auf dem die Wunde aufweisenden und die Wunde umgebenden Hautbereich. Die Vorrichtung gemäß WO1993/009727 umfasst eine Vakuumeinrichtung zur Erzeugung des Unterdrucks, eine als Dichtungseinrichtung bezeichnete luftdichte Abdeckung der Wunde, welche mit der Vakuumeinrichtung in einer funktionellen Verbindung steht, sowie eine als Schutzeinrichtung bezeichnete Wundauflage zur Positionierung an der Wunde innerhalb der Dichtungseinrichtung. Bei der Schutzeinrichtung handelt es sich um einen porösen Polymerschaumstoff, beispielsweise um Polyester-Schaum. Ausweislich der Beschreibung von WO1993/009727 kann durch die Anwendung der Unterdrucktherapie die Wundheilung unterschiedlich Typen von Wunden wie beispielsweise Brandwunden, Druckwunden oder Platzwunden beschleunigt werden.

Besonders großflächige Wunden können im Abdominalbereich entweder infolge von Verletzungen oder infolge von chirurgischen Eingriffen entstehen. Chirurgische Eingriffe im Abdominalbereich werden beispielsweise bei der operativen Behandlung akuter und lebensbedrohlicher Erkrankungen des Bauchraums vorgenommen. Im Rahmen der postoperativen Versorgung derartiger chirurgischer Eingriffe kann auch die Notwendigkeit bestehen, den offenen Abdominalbereich mittels einer temporären Abdeckung nur vorübergehend abzudecken.

Aus der WO01/85248 ist ein Verband zur temporären Abdeckung von Wunden aus Unfällen oder chirurgischen Eingriffen, insbesondere von Abdominalwunden, bekannt. Der Verband ist zur Verwendung in der Unterdrucktherapie vorgesehen. Die WO01/85248 schlägt vor, den Wundgrund mit einer mit Löchern versehenen Folie abzudecken. Auf die Folie, welche die Wundkontaktschicht darstellt, wird ein poröser Schaum aufgebracht. Auf der von der Wunde abgewandten Seite umfasst der Verband einen für Flüssigkeiten undurchlässigen Abdeckfilm mit Kleberand zum luftdichten Verschließen des Wundbereichs. Weiterhin sind Verbindungsmittel vorgesehen, welche sich durch den Abdeckfilm hindurch bis zum porösen Schaum erstrecken, um den Wundraum mit einer Unterdruckquelle verbinden zu können. Im Betrieb kann Wundexsudat aus dem Wundraum entfernt werden, indem die Flüssigkeit durch die Öffnungen der gelochten Folie zunächst zum porösen Schaum und weiterhin über den Schaum zum Verbindungsmittel gelangt, welches sich im direkten Kontakt mit dem porösen Schaum befindet.

Die WO01/852 zielt insbesondere auf einen temporären Wundverschluss, welcher ohne Beschädigungen durch Verkleben des Wundgrundes mit dem Verband gewechselt werden kann. Beschädigungen beim Verbandswechsel werden gemäß der WO01/852 vermieden, indem der direkte Kontakt porösen Materials mit dem Wundgrund vermindert wird.

Gemäß einer Ausführungsform der WO01/85248 umfasst der Verband eine oder mehrere weitere Schichten aus porösem Schaumstoff, wobei die wundseitige Schaumschicht allseitig von der mit Löchern versehenen Folie umhüllt ist.

Eine Abdominalwundauflage mit einem der WO01/85248 entsprechenden Aufbau, ist unter der Bezeichnung V.A.C. ® "Abdominal ™ Dressing Kit" (KCI) im Handel.

Von einer geschlossenen Hülle umgebene Schaumschichten können nicht auf die Größe der Wunde zugeschnitten werden, da ansonsten die Hülle zerstört wird.

Die US2009/0099519 beschreibt einen Abdominalverband für die Unterdrucktherapie, welcher ein Heizelement zur Temperaturkontrolle umfasst. Weiterhin kann der Verband gleichfalls eine von einer geschlossenen Hülle umgebene Schaumschicht (pad) umfassen, wobei die derart eingekapselte Schaumschicht in direktem Kontakt mit der Wunde stehen kann.

Eine auf die Wundkontaktschicht aufgebrachte Schaumlage, wie beispielsweise in der WO01/852 beschrieben, steht bei der Anwendung der Vorrichtung als temporärer Wundverschluss in direktem Kontakt mit den Wundrändern, da diese während der Dauer der temporären Öffnung nicht vernäht bzw. geschlossen werden. Der Kontakt zwischen Schaumlage und Wundrändern wirkt sich dabei vorteilhaft auf das Wachstum der Wundränder aus und erleichtert daher den primären Wundverschluss, wenn die Wunde nach der temporären Offenhaltung endgültig geschlossen wird.

Jedoch muss bei einer Vorrichtung, wie in der WOO1/85248 beschrieben, d.h. bei einer Abdominalwundauflage, welche neben einer mit Löchern versehene Folie als Wundkontaktschicht eine Schaumlage umfasst, jeder Kontakt der Schaumlage mit dem Wundgrund (d.h. freigelegte Organe oder Omentum majus) weitestgehend vermieden werden, um ein Verkleben oder Verwachsen des Schaums mit den freigelegten Organen oder mit dem Omentum majus zu vermeiden. Es ist daher gleichfalls ein Kontakt der Schaumlage mit dem Wundgrund durch die Öffnungen der Folie hindurch zu vermeiden. Die zwischen dem porösen Schaum und dem Wundgrund aufgebrachte Wundkontaktschicht soll daher nach der WO01/85248 weniger als 10 % Öffnungen bezogen auf die Fläche der Folie aufweisen, insbesondere nur 1 - 2 % der Fläche oder weniger. Gemäß der WO01/85248 können die Öffnungen in der Folie auch in Form von Schlitzen vorliegen, was den Kontakt zwischen Schaum und Wundgrund weiter vermindert.

Anderseits kann eine Verkleinerung der Fläche der Öffnungen in der als Wundkontaktschicht dienenden Folie zu einer verringerten Durchlässigkeit für Wundfluid führen, so dass der Abtransport von Exsudat aus dem Wundraum zur Schaumlage hin erschwert werden könnte. Während der Dauer der Applikation einer Abdominalwundauflage, insbesondere bei Verwendung einer Abdominalwundauflage als temporärer Wundverschluss, kann es nötig sein eine sehr große Flüssigkeitsmenge, beispielsweise mehr als 5 I innerhalb von 48 Stunden, abzuleiten. Sehr große abzuleitende Flüssigkeitsmengen können z.B. während der operativen Behandlung eines Darmverschlusses (Ileus) oder einer Bauchfellentzündung (Peritonitis) entstehen. Bei den aus dem Stand der Technik bekannten Abdominalwundauflagen, welche einen Verbund einer mit Löchern versehenen Folie mit einer Schaumlage umfassen, kann wie vorstehend beschrieben, die offene Fläche der Öffnungen begrenzend für den Abtransport des Exsudats sein. Darüber hinaus kann auch die Fluiddurchlässigkeit der Schaumlage begrenzend für den Abtransport des Exsudats sein: Die Fluiddurchlässigkeit der Schaumlage kann durch eine bei einer Unterdruckanwendung auftretende Kompression, welche zu einer Verringerung der Porenvolumina des Schaums führt, verringert sein. Weiterhin kann während der Dauer der Anwendung die Fluidleitfähigkeit des Schaums durch Verklumpungen weiter beeinträchtigt werden, welche von Exsudatbestandteilen verursacht werden.

Bei derzeit kommerziell erhältlichen Abdominalwundauflagen ist die Schaumlage typischerweise kleiner als die mit Öffnungen versehene Folie, so dass die Schaumlage zwar den zentralen Flächenbereich, nicht aber den Randbereich der Folie überfängt. Typischerweise wird die Schaumlage, insofern es sich um eine in Ihrer Größe anpassbare Schaumauflage handelt, in Ihrer Form an die Wundränder angepasst, so dass die Schaumlage im Kontakt mit den Wundrändern steht, während ein Randbereich der mit Öffnungen versehene Folie vorsichtig zwischen die inneren Organe und die Bauchdecke eingeschoben wird. Bei dem Einschieben der mit Öffnungen versehenen Folie zwischen die inneren Organe und der Bauchdecke muss sehr sorgfältig vorgegangen werden, um die inneren Organe nicht zu verletzen. Da in dem durch die inneren Organe und der Bauchdecke gebildeten Zwischenraum kein Hohlraum vorhanden ist, kann ein Fluidabtransport über den unter die Bauchdecke eingeschobenen Randbereich der mit Öffnungen versehenen Folie nicht erfolgen. Für die Ableitung von Wundexsudat steht deshalb nur der mit der Schaumlage überdeckte Flächenanteil der mit Öffnungen versehenen Folie zur Verfügung.

Aufgabe der vorliegenden Erfindung ist es, eine verbesserte Abdominalwundauflage für die Unterdrucktherapie in Hinblick auf eine Erhöhung von Drainagekapazität und Anwendungsfreundlichkeit zur Verfügung zu stellen. Die Wundauflage soll leicht anzulegen, gut an die Gegebenheiten der individuellen Wunde anpassbar und ohne Verklebungen bzw. Verwachsungen mit dem Untergrund zu wechseln sein.

Diese Aufgabe wird erfindungsgemäß durch eine Wundauflage gemäß Anspruch 1 gelöst. Unter Fluid werden in diesem Zusammenhang sowohl Flüssigkeiten, beispielsweise WundFluid oder Blut, als auch Gase, beispielsweise Luft oder Kohlendioxid, verstanden.

Gegenüber aus dem Stand der Technik bekannten Wundauflagen stellt die erfindungsgemäße Wundauflage weitere Hohlräume in Form biegsamer Leitungsmittel zur Ableitung von Wundexsudat zur Verfügung, wodurch die Drainagekapazität wesentlich erhöht und weiterhin über die Dauer der Anwendung stabilisiert wird. Die erfindungsgemäße Wundauflage gewährleistet eine kontinuierliche und störungsfreie Ableitung von Wundfluid über die Dauer der Anwendung der Vorrichtung, beispielsweise über einen Zeitraum von 24 bis 72 Stunden. Dabei ist vorgesehen, dass das Wundfluid aus dem Wundraum alternativ oder gleichzeitig auf unterschiedlichen Wegen zu der Unterdruckquelle gelangt. Einerseits kann das Wundfluid, bei dem es sich neben Blut beispielsweise um aus den inneren Organen ausgetretene Flüssigkeit handeln kann, aus dem Wundraum durch die Öffnungen in der ersten Folie und in den Leitungsmitteln hindurch in die Leitungsmittel gelangen. Durch die mindestens eine weitere Öffnung in den Leitungsmitteln gelangt das Fluid schließlich zu einer Unterdruckquelle. Anderseits kann Wundfluid, wie bei den im Stand der Technik bekannten Wundauflagen, aus dem Wundraum durch die mit Öffnungen versehene erste Folie hindurch zu einer Unterdruckquelle gelangen, falls die Folie nicht nur in den mit dem Leitungsmittel versehenen Flächenbereichen Öffnungen aufweist, sondern auch außerhalb dieser Bereiche. Schließlich ist es auch denkbar, dass das Wundfluid zunächst durch die Öffnungen in der ersten Folie hindurch gesaugt wird und danach durch Öffnungen in den Leitungsmitteln, falls derartige Öffnungen an geeigneter Stelle vorgesehen sind, in das Lumen der Leitungsmittel gesaugt wird. Eine kontinuierliche und störungsfreie Ableitung von Wundfluid über die Dauer der Anwendung wird dabei insbesondere über die Lumen der Leitungsmittel gewährleistet, da diese, beispielsweise im Vergleich zu einer Schaumlage, aufgrund ihrer Querschnittsgröße weniger anfällig für Verstopfungen sind.

Die vorgenannten Fluid-Verbindungen der erfindungsgemäßen Wundauflage gewährleisten neben einer Ableitung von Wundfluid aus dem Wundraum außerdem eine kontinuierliche und störungsfreie Applikation von Unterdruck (d.h. die Herstellung eines gegenüber dem Umgebungsluftdruck erniedrigten Luftdrucks) im Wundraum über die Dauer der therapeutischen Anwendung.

Die erfindungsgemäße Wundauflage kann des Weiteren anwenderseitig, also seitens des Arztes oder des medizinischen Personals, uneingeschränkt an die Form und Größe der Wunde angepasst werden, beispielsweise durch einfaches Zuschneiden mit einer sterilen Schere. Insofern es dagegen erwünscht ist, das Produkt bereits herstellerseitig an vordefinierte Wundformen und Wundgrößen angepasst bereit zu stellen, so ergeben sich durch den Aufbau der erfindungsgemäße Wundauflage in dieser Hinsicht gleichfalls keine Einschränkungen.
Insofern auf dem Verbund aus erster Folie und den darauf aufgebrachten Leitungsmitteln bei der Unterdruckbehandlung auf der von der Wunde abgewandten Seite weitere Lagen eines Verbandmaterials aufgebracht werden sollen, bietet die erfindungsgemäße Wundauflage den zusätzlichen Vorteil, dass ein Kontakt zwischen dem zusätzlichen Verbandmaterial und dem Wundgrund durch die Öffnungen der Folie hindurch weiter vermindert oder vollständig ausgeschlossen werden kann. So kann beispielsweise vorgesehen werden, dass die Öffnungen in der ersten Folie ausschließlich im Kontaktbereich von Leitungsmittel und erster Folie lokalisiert sind. In diesem Falle wird ein Kontakt zwischen dem zusätzlichen Verbandmaterial und dem Wundgrund durch die Öffnungen der Folie hindurch vollständig ausgeschlossen, während jedoch gleichzeitig Wundfluid über die Leitungsmittel zur Unterdruckquelle hin ungehindert abfließen kann. Es können auch zusätzliche Öffnungen in der ersten Folie außerhalb der Kontaktbereiche von Leitungsmitteln und erster Folie vorhanden sein. Auch in diesem Falle ist der Kontakt zwischen dem zusätzlichen Verbandmaterial und dem Wundgrund durch die Öffnungen der Folie hindurch zumindest vermindert, da die Leitungsmittel in den zwischen den Leitungsmittel gelegenen Bereichen der Folie als Abstandshalter zwischen zusätzlichem Verbandmaterial und erster Folie wirken. Ein derartiger Effekt kann durch eine geeignete Anordnung der Leitungsmittels auf der ersten Folie, beispielsweise durch eine Anbringung einer Vielzahl von Leitungsmitteln in einem dichten Abstand, verstärkt werden.

Die erfindungsgemäße Wundauflage umfasst eine erste flexible Folie mit einer ersten und einer zweiten Seite. Im Gebrauch wird die flexible Folie mit ihrer ersten Seite in direktem Kontakt auf den Wundgrund, also auf die chirurgisch oder verletzungsbedingt freigelegten inneren Organe oder das Omentum majus, gelegt. Es ist daher wesentlich, dass die erste flexible Folie aus einem Material besteht, welches während der Dauer der Anwendung nicht mit den freigelegten inneren Organen oder dem Omentum majus verklebt oder verwächst, also atraumatische Eigenschaften aufweist.

Geeignete Materialien für die erste flexible Folie umfassen thermoplastische Folien für Medizinzwecke, insbesondere Folien aus PU, PE, PET, PVC, Silikon oder einer Mischung daraus. Vorzugsweise handelt es sich bei der thermoplastischen Folie um eine PU-Folie. Gemäß einer besonders bevorzugten Ausführungsform handelt es sich bei der ersten flexiblen Folie um eine Stützfilm-freie PU-Folie mit einer Dicke von 50 bis 60 µm, beispielsweise das Produkt "Folie 4142 medical transparent" der Firma Gerlinger Industries (Nördlingen, Deutschland).

Die erste flexible Folie weist eine Vielzahl von Öffnungen auf, welche einen Durchtritt von Wundfluid ermöglichen. Bei den Öffnungen kann es sich um Löcher oder um Schlitze handeln. Hinsichtlich Form und Größe der Öffnungen kann in geeigneter Weise auf die erwünschte Durchlässigkeit der Folie für Wundfluid abgestellt werden. Denkbar sind runde, ovale, eckige oder beispielsweise sternförmige Löcher. Bei den Schlitzen kann es sich beispielsweise um längliche oder kreuzförmige Schlitze handeln.

Falls es sich bei den Öffnungen um Schlitze handelt, sollten diese eine Länge von jeweils mindestens 1 mm und höchstens 30 mm, vorzugsweise mindestens 2 mm und höchstens 20 mm und insbesondere mindestens 5 mm und höchstens 10 mm aufweisen. Dabei hat sich gezeigt, dass insbesondere eine Folie, welche Schlitze mit einer Länge von mindestens 5 mm und höchstens 10 mm aufweist, wobei zwischen 10 und 90 derartiger Schlitze auf einer Folien-Fläche von 100 cm² eingebracht werden, besonders vorteilhafte Eigenschaften hinsichtlich Stabilität bei gleichzeitig ausreichender Fluid-Durchlässigkeit aufweist.

Falls es sich bei den Öffnungen dagegen um Löcher handelt, so kann deren Durchmesser hinsichtlich der erwünschten Fluid-Durchlässigkeit in geeigneter Weise angepasst werden, beispielsweise in einem Bereich vom 0,1 mm bis 20 mm. Bereiche zwischen mindestens 0,2 mm und höchstens 10 mm, insbesondere mindestens 0,3 mm und höchstens 5 mm, sind dabei hinsichtlich einer günstigen Kombination von Fluid-Durchlässigkeit und atraumatischen Eigenschaften besonders bevorzugt.

Die Öffnungen können gleichmäßig, d.h. in regelmäßig sich wiederholenden Mustern oder zufällig über die Fläche der ersten Folie verteilt vorliegen. Es ist jedoch auch möglich die Öffnungen nur in bestimmten Flächenbereichen anzubringen. Insbesondere ist es bevorzugt, die Öffnungen nur in denjenigen Flächenbereichen der ersten Folie anzubringen, welche im Kontakt mit dem Leitungsmittel stehen, wenn auf dem Verbund aus Folie und Leitungsmittel weiteres Verbandsmaterial aufgebracht werden soll. Ein Kontakt zwischen Verbandsmaterial und Wundgrund wird durch eine solche Anordnung ausgeschlossen.
Gemäß einer weiteren Ausführungsform befinden sich die Öffnungen nur im zentralen Bereich der ersten Folie, während ein Randbereich der Folie frei von Öffnungen ist. Eine umgekehrte Anordnung ist gleichfalls möglich.

Falls es sich bei den Öffnungen in der ersten Folie um Löcher handelt, soll die Summe der offenen Fläche der Löcher mindestens 0,2 %, vorzugsweise mindestens 1 % der Flächenerstreckung der Folie betragen, um eine ausreichende Durchlässigkeit der Folie für Wundfluid zu gewährleisten. Dabei soll möglichst eine offene Fläche von 20 % der Folie nicht überschritten werden, da sich dies andernfalls negativ auf die Stabilität der ersten Folie auswirken könnte. Vorzugsweise umfasst die erste Folie Löcher, welche weniger als 15 % der Folie einnehmen.

Denkbar ist auch eine Kombination von Löchern und Schlitzen, wobei es möglich ist, dass bestimmte Flächenbereiche der ersten Folie Löcher aufweist und ein anderer Flächenbereich der ersten Folie Schlitze aufweist. So kann es vorteilhaft sein, diejenigen Flächenbereiche der ersten Folie, welcher mit einem Leitungsmittel in Kontakt steht, mit Öffnungen zu versehen, während ein davon unterschiedlicher Flächenbereich, welcher zwischen den Leitungsmitteln angeordnet ist, mit Schlitzen zu versehen. Eine derartige Anordnung von Löchern und Schlitzen kann insbesondere dann wünschenswert sein, wenn auf dem Verbund aus Folie und Leitungsmittel weiteres Verbandsmaterial aufgebracht wird, da hierdurch der Kontakt von Verbandmaterial und Wundgrund (innere Organe oder das Omentum majus) minimiert oder völlig verhindert wird.

Erfindungsgemäß sind hinsichtlich Form, Größe, Anzahl und Anordnung der Öffnungen in der ersten flexiblen Folie weitere, hier nicht speziell aufgeführte Varianten umfasst.

Die Öffnungen können vor dem Befestigen der Leitungsmittel in die erste Folie eingebracht werden. Es ist jedoch auch möglich die Öffnungen erst nach dem Befestigen der Leitungsmittel auf die erste Folie anzubringen. Die letztgenannte Variante ist insbesondere dann vorteilhaft, wenn ausschließlich derjenige Flächenbereich der ersten Folie Öffnungen aufweisen soll, welcher mit einem Leitungsmittel in Kontakt ist.
Gemäß einer weiteren Ausführungsform können in die erste Folie Schlitze eingebracht werden, bevor das Leitungsmittel auf der Folie befestigt wird. Nach dem Befestigen der Leitungsmittels auf der ersten Folie können dann Löcher ausschließlich in denjenigen Flächenbereich der ersten Folie Öffnungen eingebracht werden, welcher mit einem Leitungsmittel in Kontakt ist.

Die mindestensdrei, auf der ersten Folie aufgebrachten Leitungsmittel befinden sich auf der von der Wunde abgewandten zweiten Seite der ersten Folie. Typischerweise wird der Rand des Verbundes aus erster Folie und Leitungsmittel nach dem Auflegen vorsichtig zwischen die Bauchdecke und den Wundgrund eingeschoben. Es kann aber auch hinsichtlich der Dicke der Wundauflage vorteilhaft sein, dass die mindestens drei Leitungsmittel lediglich im zentralen Bereich der ersten Folie aufgebracht sind, so dass ein Randbereich der ersten Folie vorhanden ist, welcher keine aufgebrachten Leitungsmittel aufweist und insbesondere zum Einschieben zwischen die Bauchdecke und den Wundgrund vorgesehen ist.

Der Verbund aus erster Folie und Leitungsmittel soll insgesamt über eine Flexibilität und Weichheit verfügen, so dass sich die Wundauflage dem Wundgrund dicht anschmiegt. Flexibilität und Weichheit des Verbundes aus erster Folie und Leitungsmitteln werden insbesondere durch die Eigenschaften der Einzelkomponenten Folie und Leitungsmittel bestimmt. Daher ist es wesentlich, dass auch die auf der ersten Folie aufgebrachten Leitungsmittel aus einem biegsamen elastomeren Material bestehen. Gemäß einer bevorzugten Ausführungsform sollten die Leitungsmittel deshalb aus einem Material gefertigt sein, welches eine Shore A Härte von höchstens 60 aufweist (bestimmt nach DIN 53505 vom August 2000, und zwar bei 23°C an einem wie in der Norm beschrieben plattenförmigen ebenen und glatten Probekörper einer Dicke von 6 mm). In besonders vorteilhafter Weise werden für die Leitungsmittel noch weichere Materialien, das heißt Materialien mit einer Shore A Härte von weniger als 50, insbesondere mit einer Shore A Härte von weniger als 45, besonders bevorzugt mit einer Shore A Härte von weniger als 40 eingesetzt. Zudem kann ein Verbund aus erster Folie und Leitungsmitteln besonders leicht zugeschnitten werden, wenn die Shore A höchstens 60, vorzugsweise weniger als 50, besonders bevorzugt weniger als 45, insbesondere weniger als 40 beträgt. Dies erleichtert die Anpassung der Wundauflage an die Wundform.

Bei den Leitungsmitteln handelt es sich um Drainageschläuche für medizinische Zwecke, wobei die Drainageschläuche PU (Polyurethan), PVC (Polyvinylchlorid), PE (Polyethylen), PET (Polyethylenterephthalat), PTFE (Polytetrafluorethylen), Silikon oder eine Mischung daraus umfassen.

Weiterhin ist es hinsichtlich der Flexibilität des Verbundes aus erster Folie und Leitungsmitteln wesentlich, dass die Leitungsmittel eine Dickenerstreckung (H) von höchstens 30 mm, vorzugsweise weniger als 20 mm, insbesondere von weniger als 15 mm, aufweisen.

Die Leitungsmittel sollten jeweils eine Vielzahl von Öffnungen aufweisen, welche einen Durchtritt von Wundfluid aus dem Wundraum, insbesondere aus der Bauchhöhle, in das Lumen der Leitungsmittel ermöglichen. Hierbei ist es vorteilhaft, dass wenigstens ein ausreichender Anteil der Öffnungen der Leitungsmittel über den Öffnungen in der ersten Folie zu liegen kommt, so dass Flüssigkeit aus dem Wundraum direkt in das Lumen der Leitungsmittel übertreten kann. Bei den Öffnungen in den Leitungsmitteln handelt es sich um Löcher, wobei hinsichtlich Form, Anordnung und Größe keine grundsätzlichen Einschränkungen bestehen. Vorzugsweise handelt es sich um runde, ovale oder eckige Löcher, wobei die Löcher eine offene Fläche von jeweils wenigstens 0,5 mm² aufweisen sollen.

Weiterhin weisen die Leitungsmittel mindestens eine weitere, endständige Öffnung auf, die eine Fluid-Kommunikation mit einer Unterdruckquelle ermöglicht.

Die auf der ersten Folie aufgebrachten Leitungsmittel weisen hinsichtlich ihres äußeren Umfangs vorzugsweise eine insgesamt flache Form auf. Unter einer insgesamt flachen Form der Leitungsmittel wird im Zusammenhang mit der Erfindung verstanden, dass die Leitungsmittel ein Verhältnis ihrer Querausdehnung bzw. Breite (Durchmesser des Leitungsmittel in einer zur ersten Folie parallel liegenden Ebene) zu ihrer Dickenerstreckung bzw. Höhe (H; Durchmesser des Leitungsmittel in einer zur ersten Folie senkrecht stehenden Ebene) von mehr als 1,25, vorzugsweise mehr als 1,5, aufweisen, wobei die Dickenerstreckung (H) höchstens 30 mm, vorzugsweise weniger als 20 mm, insbesondere weniger als 12 mm, aufweist. Gemäß einer besonders bevorzugten Ausführungsform der Erfindung beträgt dabei die Breite zwischen 8 mm und 40 mm, insbesondere zwischen 15 und 35 mm.
Gemäß einer Variante (Möglichkeit a gemäß Anspruch 1) der erfindungsgemäßen Wundauflage sind die Leitungsmittel mit wenigstens 70 %, vorzugsweise 80 %, ihrer senkrecht auf die erste Folie projizierten Fläche mit der zweiten Seite der ersten Folie unlösbar und flächenhaft verbunden. Besonders bevorzugt handelt es sich bei dem Leitungsmittel um einen Drainageschlauch mit einem insgesamt im Wesentlichen rechteckigem äußerem Umfang, bei welchem mindestens 90 % seiner senkrecht auf die erste Folie projizierten Fläche mit der zweiten Seite der ersten Folie unlösbar und flächenhaft verbunden werden kann.

Die insgesamt flache Form ist einerseits vorteilhaft, um eine im Gebrauch stabile und gegebenenfalls unlösbare Verbindung mit der ersten Folie zu gewährleisten. Anderseits wird durch die flache Form eine ausreichend große Kontaktfläche zur Verfügung gestellt, so dass eine Vielzahl von durch erste Folie und zweite Folie reichende Öffnungen vorgesehen werden kann.

Die Leitungsmittel weisen ein Lumen in Form einer oder mehrerer durchgehender Hohlräume auf, um eine Ableitung von Wundfluid zur Unterdruckquelle hin, sowie einen Unterdruck im Wundraum zu gewährleisten. Entsprechend können die Leitungsmittel entweder nur einen Hohlraum, oder aber mehrere Hohlräume umfassen, um das Lumen zu bilden.

Falls die Leitungsmittel mehrere Hohlräume aufweisen, so können die einzelnen Hohlräume über Öffnungen miteinander verbunden sein, um ein kontinuierliches Lumen zu bilden. Es ist jedoch auch denkbar, dass die Leitungsmittel mehrere durchgehend verlaufende Hohlräume aufweisen, welche nicht miteinander in Verbindung stehen. Dabei sollte die gesamte offene Querschnittsfläche des Lumens mindestens 1 mm², vorzugsweise mindestens 5 mm², betragen, um eine ausreichende Durchflusskapazität für die Ableitung von Wundfluid bereit zu stellen. Insbesondere soll das Lumen des Leitungsmittels eine Querschnittsfläche von insgesamt 10 bis 50 mm², vorzugsweise 20 bis 35 mm² aufweisen. Unter "gesamter offener Querschnittsfläche des Lumens" wird in diesem Zusammenhang die Summe der offenen Querschnittsflächen der einzelnen Hohlräume eines einzelnen Leitungsmittels verstanden, wobei sich das Leitungsmittel in einem nicht-zusammengedrückten Zustand befindet. Vorzugsweise umfasst das Leitungsmittel nur zwei oder drei durchgehende Hohlräume, welche insbesondere miteinander Fluid-leitend verbunden sind.
Gemäß einer weiteren besonders bevorzugten Ausführungsform weisen die Leitungsmittel nur einen einzigen durchgehenden Hohlraum auf.

Weiterhin müssen die Leitungsmittel über eine ausreichende Unterdruckstabilität verfügen. Der Ausdruck "Unterdruck im Wundraum" bezeichnet im Zusammenhang mit der Erfindung einen innerhalb des Wundverbandes gegenüber dem Umgebungsluftdruck (atmosphärischer Luftdruck) erniedrigten Luftdruck. Unter "innerhalb des Wundverbandes" wird der durch das Abdeckmaterial und der Wunde gebildete Zwischenraum verstanden. "Unterdruck" wird häufig auch als "negativer Druck" bezeichnet.

Die Druckdifferenz zwischen dem Luftdruck innerhalb des Wundverbandes und dem Umgebungsluftdruck wird im Zusammenhang mit der Erfindung in mm Hg (Millimeter-Quecksilbersäule) angegeben, da dies im Bereich der Unterdrucktherapie üblich ist. 1 mm Hg entspricht einem Torr beziehungsweise 133,322 Pa (Pascal). Im Zusammenhang mit der Erfindung wird der Unterdruck, d.h. die Druckdifferenz zwischen dem Luftdruck innerhalb des Wundverbandes und dem Umgebungsluftdruck, als positiver Zahlenwert in mm Hg angegeben. Unter einer ausreichenden Unterdruckstabilität wird verstanden, dass die Leitungsmittel bei einem in dem bei der Unterdrucktherapie eingesetzten Unterdruck von typischerweise 125 mmHg einen offenen Querschnitt von mindestens 1 mm² behalten. Ein unerwünschtes Zusammendrücken der Leitungsmittel im Unterdruck kann auf unterschiedliche, dem Fachmann bekannte Weise, verhindert werden, beispielsweise durch eine geeignete Querschnittsgeometrie und/oder durch Auswahl geeigneter Materialien. So können in den Leitungsmitteln beispielsweise asymmetrisch angeordnete Innenwandrippen vorgesehen sein, welche sich vorteilhaft auf die Unterdruckstabilität des Leitungsmittels auswirken. Erfindungsgemäß geeignete Leitungsmittel mit einer ausreichenden Unterdruckstabilität sind auch als Fertigprodukt in für medizinische Anwendungen geeigneter Qualität kommerziell erhältlich. Derartige Leitungsmittel werden üblicherweise als Drainageschläuche bezeichnet. So bietet beispielsweise die Firma Primed (Halberstadt, Deutschland) flache Drainageschläuche in unterschiedlichen Abmessungen unter der Bezeichnung "J.P. Flachdrain" an.

Gemäß einer besonders vorteilhaften Ausführungsform weisen die Leitungsmittel eine Beschichtung mit einer antikoagulierend wirkenden Substanz (beispielsweise Heparin, oder eine andere, üblicherweise zur Beschichtung von medizinischen Schläuchen oder Röhrchen verwendete Substanz mit antikoagulierender Wirkung) auf. Mittels einer derartigen antikoagulierenden Beschichtung kann einer Verstopfung der Leitungsmittel durch Blutklumpen vorgebeugt werden, so dass ein gleichmäßig hoher Durchfluss von Wundexsudat über eine längere Anwendungsdauer gewährleistet bleibt. Mit einem Antikoagulans beschichtete medizinische Drainageschläuche sind als Fertigprodukt kommerziell erhältlich, beispielsweise von der Firma Axiom Medical (Bürstadt, Deutschland).

Erfindungsgemäß ist vorgesehen, dass mindestens drei Leitungsmittel auf die zweite Seite der ersten Folie aufgebracht werden.

Die Wundauflage umfasst mindestens drei Leitungsmittel, d.h. mindestens drei separate Einheiten des Leitungsmittels, welche gemäß einer ersten erfindungsgemäßen Variante in einer sternförmigen Anordnung auf der ersten Folie aufgebracht werden.
Alternativ können mehrerer Leitungsmittel gemäß einer zweiten erfindungsgemäßen Variante in weitestgehend paralleler Anordnung auf die erste Folie aufgebracht werden. Dabei können zwischen den parallelen Strängen weitere Einheiten des Leitungsmittels als Querverbindungen vorgesehen sein. Entsprechend umfasst die Erfindung eine Wundauflage, wobei mindestens drei Leitungsmittel in weitestgehend paralleler Anordnung auf die zweite Seite der ersten Folie aufgebracht sind, und wobei weiterhin Querverbindungen zwischen den Leitungsmitteln vorgesehen sind, so dass mittels der Querverbindungen eine Fluidkommunikation zwischen den Leitungsmitteln hergestellt werden kann.

Die Leitungsmittel werden auf die zweite Seite der ersten Folie aufgebracht. Darunter wird im Zusammenhang mit der vorliegenden Erfindung verstanden, dass entweder a) die Leitungsmittel mit der zweiten Seite der ersten Folie unlösbar und flächenhaft verbunden werden oder b) dass das Leitungsmittel auf der zweiten Seite der ersten Folie durch Befestigungsmittel an der Folie gehalten wird.

Im Falle der ersten Möglichkeit a) sollen dabei die Leitungsmittel mit wenigstens 70 % ihrer senkrecht auf die erste Folie projizierten Fläche mit der zweiten Seite der ersten Folie unlösbar und flächenhaft verbunden sein, da auf diese Weise eine ausreichende Stabilität und Festigkeit des Verbundes aus erster Folie und Leitungsmitteln gewährleistet wird.

Im Falle der zweiten Möglichkeit b) sollen dabei dabei die Leitungsmittel mit wenigstens 85 % ihrer senkrecht auf die erste Folie projizierten Fläche auf der zweiten Seite der ersten Folie aufliegen, wobei die Leitungsmittel durch Befestigungsmittel auf der ersten Folie gehalten werden. Unter Befestigungsmittel wird dabei eine Vielzahl dem Fachmann bekannter Befestigungsmittel verstanden, beispielsweise Klebestreifen oder Klammern. Insbesondere ist es auch möglich eine weitere Schicht, insbesondere eine weitere flexible Folie, dergestalt aufzubringen, dass die Leitungsmittel zwischen erster und weiterer Folie stabil gehalten werden. Eine derartig aufgebrachte weitere Folie, welche die Leitungsmittel beispielsweise in Form einer Tasche auf der ersten Folie hält, wird von dem Begriff Befestigungsmittel umfasst. Es ist im Zusammenhang mit dieser Ausführungsform bevorzugt, dass keine Verklebung zwischen den Leitungsmitteln und einer der beiden Folien vorliegt. Stattdessen liegt eine unlösbare Verbindung zwischen erster und weiterer Folie vor.

Gemäß einer besonders vorteilhaften Ausgestaltung der Erfindung umfasst die Wundauflage eine flexible Leitungsplatte, welche zur Aufnahme eines Endbereichs der mindestens drei Leitungsmittel geeignet ist. Die Leitungsplatte wird vorzugsweise im mittigen Bereich der im Gebrauch von der Wunde abgewandten Seite der Verbundes aus erster Folie und Leitungsmitteln oder - falls vorhanden - des Verbundes aus erster Folie, Leitungsmittel und zweiter Folie, angebracht oder aufgelegt. Die Leitungsplatte umfasst zweckmäßigerweise einen medizinisch geeigneten Kunststoff, beispielsweise aus PU (Polyurethan), PVC (Polyvinylchlorid), PE (Polyethylen), PET (Polyethylenterephthalat), PTFE (Polytetrafluorethylen), Silikon oder eine Mischung daraus. Dabei ist es hinsichtlich der Anwendung vorteilhaft, wenn die Leitungsplatte insgesamt weich und flexibel ausgebildet ist. Vorzugsweise sollte die Leitungsplatte aus einem elastomeren Material bestehen, welches eine Shore A Härte von höchstens 80, vorzugsweise höchstens 70 und insbesondere von höchstens 60 aufweist. Die Leitungsplatte kann unlösbar auf der zweiten Seite der ersten Folie befestigt sein. Dabei ist insbesondere vorgesehen, dass die Leitungsplatte dergestalt auf dem Verbund aus Folie(n) und Leitungsmitteln befestigt wird, dass sich es während der Dauer der Unterdrucktherapie unter einem Unterdruckanschlussstück befindet. Die Leitungsplatte sollte möglichst hinsichtlich ihrer flächenhaften Ausdehnung auf das Unterdruckanschlussstück abgestimmt sein. Darunter wird verstanden, dass die Leitungsplatte eine auf eine Unterlage projizierte Fläche aufweist, welche der auf eine Unterlage projizierten Fläche des Unterdruckanschlussstücks weitgehend entspricht. Die auf eine auf eine Unterlage projizierten Flächen von Leitungsplatte und Unterdruckanschlussstücks sollten idealerweise weniger als 50 % Abweichung voneinander aufweisen. Eine derartige Anordnung trägt zur Stabilisierung der Wundauflage bei und kann zur Verminderung unerwünschter mechanischer Einwirkungen auf den Wundgrund beitragen.

Gemäß einer einfachen Ausführung dient die Leitungsplatte lediglich zur korrekten Positionierung der Leitungsmittel im Sinne einer Auflageplatte. Bei dieser Ausführungsform werden die Leitungsmittel, welche im Bereich der Leitungsplatte eine Öffnung aufweisen, an der Leitungsplatte befestigt.

Gemäß einer weiteren Ausführung bildet die Leitungsplatte einen zentralen Hohlraum in Sinne einer flachen Dose, wobei der zentrale Hohlraum mit den Lumina der Leitungsmittel über eine Öffnung, insbesondere über ein offenes Ende des Leitungsmittels, Fluid-leitend verbunden ist. Der Hohlraum der Leitungsplatte weist auf der von der Wunde abgewandten Seite Durchgänge auf, um eine Fluid-Kommunikation mit dem Unterdruckanschlussstück herstellen zu können.
Die Leitungsplatte kann rund, quadratisch oder polygonal ausgebildet sein und weist einen Durchmesser von mindestens 1 cm und höchstens 12 cm, insbesondere mindestens 2 cm und höchstens 10 cm auf.

Gemäß einer sehr vorteilhaften Ausführungsform der Erfindung umfasst die Wundauflage weiterhin eine zweite flexible Folie, wobei die zweite Folie auf die im Gebrauch von der Wunde abgewandte Seite des Verbundes aus erster Folie und Leitungsmitteln aufgebracht wird. Vorzugsweise ist die zweite Folie aus einem weitestgehend Fluid-undurchlässigem Material gefertigt. Zur Herstellung der zweiten flexiblen Folie können insbesondere die vorstehend bereits im Zusammenhang mit der ersten flexiblen Folie erwähnten Materialien und Fertigprodukte verwendet werden, beispielsweise thermoplastische Folien für Medizinzwecke, insbesondere Folien aus PU, PE, PET, PVC, Silikon oder einer Mischung daraus.

Gemäß einer Ausführungsform kann die zweite Folie eine Vielzahl von über die Fläche verteilten Öffnungen aufweisen.
Vorzugsweise ist die zweite Folie jedoch weitestgehend Fluid-undurchlässig ausgebildet und weist lediglich eine einzige, vorzugsweise im zentralen Bereich angeordnete, Öffnung auf. Die zweite Folie wird dabei unlösbar auf dem Verbund aus erster Folie und Leitungsmitteln befestigt, beispielsweise durch Verkleben oder durch thermisches Verschweißen. Als Befestigungsstellen kommen dabei entweder die Leitungsmittel und/oder diejenigen Bereiche der ersten flexiblen Folie in Frage, welche zwischen den Leitungsmitteln lokalisiert sind.

Die von der erfindungsgemäßen Wundauflage optional umfasste zweite flexible Folie kann in vorteilhafter Weise dazu beitragen die Stabilität und Handhabbarkeit der Wundauflage zu verbessert. Weiterhin können durch eine geeignete Kombination von erster flexibler Folie und zweiter flexibler Folie die Durchlässigkeit der Wundauflage für Wundfluid, sowie die atraumatischen Eigenschaften der Wundauflage in geeigneter Weise modifiziert und optimiert werden. Dies kann zu einer verbesserten und beschleunigten Wundheilung beitragen, da Wundfluid effizient aus dem Wundraum entfernt werden kann und Schädigungen des Wundgrunds, bzw. der freigelegten inneren Organe oder des Omentum majus vermindert werden.

Gemäß einer Ausführungsform der Wundauflage weist die zweite Folie eine Vielzahl von über die Fläche verteilten Öffnungen auf, welche zum Durchleiten von Fluid geeignet sind. Dabei kann es sich als vorteilhaft erweisen, wenn die in der ersten Folie vorhandenen Öffnungen und die in der zweiten Folie vorhandenen Öffnungen so angeordnet sind, dass die Öffnungen weitestgehend nicht zueinander in Deckung stehen. Unter "weitestgehend nicht zueinander in Deckung stehend" wird in diesem Zusammenhang verstanden, dass zueinander in Deckung stehende Öffnungen von erster und zweiter Folie höchstens zufällig und nur in geringer Anzahl vorhanden sind. Insbesondere sollen dabei mindestens 90% vzw. 95% der Öffnungen in der ersten Folie nicht mit einer Öffnung in der zweiten Folie in Deckung stehen.

Gemäß einer weiteren bevorzugten Ausführungsform ist die zweite Folie weitestgehend fluidundurchlässig ausgebildet und weist lediglich eine einzige, vorzugsweise im zentralen Bereich angeordnete Öffnung auf.

Erfindungsgemäß kann die zweite Folie auf unterschiedliche Weise auf dem Verbund aus erster Folie und Leitungsmittel angeordnet sein. Beispielhafte Ausführungsformen werden im Folgenden dargelegt, wobei weitere Anordnung denkbar sind und von der Erfindung umfasst sind.

Gemäß einer ersten Ausführungsform wird die zweite Folie auf die mindestens drei Leitungsmittel geklebt, so dass ein stabiler Verbund aus erster Folie, Leitungsmitteln und zweiter Folie entsteht. Dies ist technisch leicht zu realisieren, bspw. in dem die von der ersten Folie wegweisenden Seiten der Leitungsmittel an ihrer Oberfläche mit einem Kleber beschichtet werden und die zweite flexible Folie auf die derart vorbereiteten Leitungsmittel gelegt wird.

Gemäß einer weiteren Ausführungsform ist die zweite Folie mit der ersten Folie direkt verklebt, jedoch nicht mit den Leitungsmitteln. Die Verklebung von erster Folie und zweiter Folie kann dabei flächig, punktförmig oder linienförmig erfolgen. Es ist auch denkbar, dass erste Folie und zweite Folie nicht verklebt, sondern durch Verschweißen miteinander in eine unlösbare Verbindung gebracht werden. Das Schweißen kann dabei insbesondere punktförmig oder entlang von Schweißnähten erfolgen. Eine direkte, unlösbare Verbindung von erster und zweiter Folie ergibt eine besonders stabile Anordnung.

Gemäß einer weiteren Ausführungsform ist es auch möglich, die zweite Folie mit der ersten Folie und mit den Leitungsmitteln zu verkleben. Auf diese Weise ergibt sich ein besonders stabiler Verbund.

Gemäß einer besonders vorteilhaften Ausführungsform erfolgt die Befestigung der zweiten Folie auf der ersten Folie durch punktförmiges Verkleben oder Verschweißen in den Bereichen außerhalb der Leitungsmittel. Dabei kann insbesondere eine Vielzahl von über die Fläche verteilten Haftpunkten vorgesehen sein. Bei einer derartigen Verbindung von erster und zweiter Folie entsteht ein Fluid-leitender Zwischenraum zwischen den Folien, welcher den Abfluss von Wundexsudat in vorteilhafter Weise begünstigen kann. Dabei ist bevorzugt, dass die erste Folie über eine Vielzahl von über die gesamte Fläche verteilten Öffnungen aufweist und weiterhin die Leitungsmittel über seitliche Öffnungen verfügen. Wundexsudat aus dem Zwischenraum, welcher durch erste und zweite Folie gebildet wird, kann dabei über die seitlichen Öffnungen der Leitungsmittel abgesaugt werden. Bei der hier genannten besonders vorteilhaften Ausgestaltung der Erfindung befinden sich Leitungsmittel und - falls vorhanden - Leitungsplatte in einer Tasche zwischen den beiden Folien, und werden auf diese Weise auf der ersten Folie gehalten. Die Verschweißung von erster und zweiter Folie dient hierbei als Befestigungsmittel für die Leitungsmittel, so dass darauf verzichtet werden kann die Leitungsmittel mit der ersten Folie zu verkleben. Hierbei sollten die mindestens drei Leitungsmittel mit wenigstens 85 % ihrer senkrecht auf die erste Folie projizierten Fläche auf der zweiten Seite der ersten Folie aufliegen.

Bei der Anwendung der Wundauflage zur temporären Abdeckung großflächiger Wunden des Abdominalbereichs wird üblicherweise der Randbereich der ersten Folie oder der Randbereich des Verbundes aus erster Folie, Leitungsmitteln und - falls vorhanden - zweiter Folie, zwischen die Bauchdecke und den Wundgrund oder auf das Omentum majus eingebracht. Dazu wird typischerweise ein flaches chirurgisches Instrument, beispielsweise ein Bauch- oder Darmspatel, verwendet. Die Applikation des Randbereichs der Wundauflage zwischen Bauchdecke und den Wundgrund stellt große technische Anforderung an den Chirurgen und geht mit einer Verletzungsgefahr der inneren Organe einher. Dabei hat es sich insbesondere als schwierig erwiesen, den Randbereich der Wundauflage faltenfrei unter die Bauchdecke zu applizieren. Es wurde nun gefunden, dass die Wundauflage wesentlich sicherer, schneller und gleichzeitig weitgehend faltenfrei in den Bereich zwischen die Bauchdecke und den Wundgrund eingebracht werden kann, wenn die Wundauflage auf der Gebrauch von der Wunde abgewandte Seite des Verbundes aus erster Folie und Leitungsmittel oder des Verbundes aus erster Folie, Leitungsmitteln und zweiter Folie mindestens eine oder mehrere randständige und zur Mitte der Wundauflage hin offene Taschen mit einer Tiefe von höchstens 6 cm aufweist. Der behandelnde Arzt kann den Bauch- oder Darmspatel in die Taschen einführen und sodann den Randbereich der Wundauflage zwischen Bauchdecke und den Wundgrund einbringen.

Derartige Taschen, welche das gleichmäßige Aufbringen und Auslegen des Verbundes auf die freigelegten inneren Organe oder auf das Omentum majus erleichtern, können insbesondere durch Einfalten der ersten und/oder zweiter Folie oder durch das Aufbringen von zusätzlichen Folienstücke gebildet werden.

Entsprechend umfasst die vorliegende Erfindung gemäß einer bevorzugten Ausführungsform eine Wundauflage, welche auf der Gebrauch von der Wunde abgewandte Seite des Verbundes aus erster Folie und Leitungsmitteln oder des Verbundes aus erster Folie, Leitungsmitteln und zweiter Folie mindestens eine oder mehrere randständige und zur Mitte der Wundauflage hin offene Taschen mit einer Tiefe von höchstens 6 cm aufweist, welche das gleichmäßige Aufbringen und Auslegen des Verbundes auf die freigelegte inneren Organe oder auf das Omentum majus erleichtern, wobei die Taschen vorzugsweise durch Einfalten der ersten und/oder zweiter Folie und/oder durch das Aufbringen von Folienstücke gebildet werden. Insbesondere sind die eine oder mehreren Taschen auf mindestens 50 % des Umfangs des Verbundes aus erster Folie und Leitungsmittel oder des Verbundes aus erster Folie, Leitungsmittel und zweiter Folie vorgesehen.

Es hat sich weiterhin als sehr vorteilhaft erwiesen, wenn die Wundauflage auf der von der Wunde weg weisenden Seite mindestens eine weitere flüssigkeitsdurchlässige Schicht umfasst. Gemäß einer weiteren Ausführungsform der Erfindung umfasst die Wundauflage deshalb mindestens eine weitere flüssigkeitsdurchlässige Schicht zum Aufbringen auf die im Gebrauch von der Wunde abgewandte Seite des Verbundes aus erster Folie und Leitungsmitteln oder des Verbundes aus erster Folie, Leitungsmitteln und Leitungsplatte oder des Verbundes aus erster Folie, Leitungsmitteln und zweiter Folie oder des Verbundes aus erster Folie, Leitungsmitteln, Leitungsplatte und zweiter Folie. Die flüssigkeitsdurchlässige Schicht umfasst vorzugsweise einen porösen Schaumstoff, insbesondere einen porösen Polymerschaumstoff. Besonders geeignet sind dabei offenzellige Polymerschaumstoffe. Im Rahmen dieser Anmeldung bedeutet der Begriff offenzellig, dass im Schaumstoff (c) mindestens 60 % offene Zellen, bevorzugt mindestens 90 % offene Zellen, mehr bevorzugt mindestens 98 % offene Zellen, insbesondere im Wesentlichen 100 % offene Zellen, bezogen auf die Gesamtzahl an Zellen, vorhanden sind.
Geeignete Materialien für einen porösen Schaumstoff umfassen beispielsweise Polyurethan, Polyurethan-Polyharnstoff-Copolymere, Polyvinylalkohol (PVA) oder Silikon.

Alternativ oder zusätzlich kann die flüssigkeitsdurchlässige Schicht für Medizinzwecke geeignete textile Materialien wie Woven oder Non-woven umfassen, beispielsweise einen Vliesstoff aus synthetischen Polymeren wie Polyamid, Polyester oder Polypropylen.

Im Zusammenhang mit der vorliegenden Erfindung können die in der deutschen
Patentanmeldung DE102010034819 beschriebenen porösen Schaumstoffe in besonders vorteilhafter Weise zur Herstellung der einen oder mehreren flüssigkeitsdurchlässigen Schichten eingesetzt werden. Auf den Inhalt der deutschen Patentanmeldung DE102010034819.8 wird hiermit Bezug genommen. Die in der DE102010034819.8 beschriebenen Schaumstoffe setzen beim zur Anpassung an die Wundform gegebenenfalls nötigen Zuschneiden keine oder nur im geringen Maße Schaumstoffpartikel frei. Freigesetzte Schaumstoffpartikel, welche in die Wunde gelangen, können die Wunde reizen und die Wundheilung beeinträchtigen.

In besonders vorteilhafter Weise kann der vom Anmelder Paul Hartmann AG (Heidenheim, Deutschland) vertriebene offenzellige Polyurethan-Schaumstoff VivanoMed als flüssigkeitsdurchlässige Schicht eingesetzt werden.

Die mindestens eine weitere flüssigkeitsdurchlässige Schicht kann die Weichheit und Verträglichkeit der Wundauflage verbessern und einen zusätzlichen Beitrag zur Ableitung von Wundexsudat leisten.

Weiterhin ist es zur Förderung des primären Wundverschlusses insbesondere sehr vorteilhaft einen porösen Polymerschaumstoff derart anzubringen, dass der Schaumstoff in direktem Kontakt mit den Wundrändern steht.

Die mindestens eine weitere flüssigkeitsdurchlässige Schicht weist eine Dicke von 2 mm bis 50 mm, vorzugsweise von 3 mm bis 30 mm auf.

Die weitere flüssigkeitsdurchlässige Schicht, welche vorzugsweise einen porösen Polymerschaumstoff umfasst, kann dabei die gesamte Fläche des Verbundes aus Leitungsmitteln und erster Folie (oder falls vorhanden des Verbundes aus erster Folie, Leitungsmitteln und zweiter Folie) überfangen. Es ist jedoch auch möglich, dass die flüssigkeitsdurchlässige Schicht nur auf einem Teil der Fläche von erster oder - falls vorhanden - zweiter Folie vorliegt. Insbesondere bei der Verwendung der Wundauflage als temporärer Wundverschluss hat es sich als günstig erwiesen, wenn ein poröser Polymerschaumstoff derart auf die erste oder - falls vorhanden - zweite Folie aufgebracht wird, dass die Ränder des Schaumstoffs in direktem Kontakt mit den Wundrändern stehen, während die erste Folie oder der Verbund aus Folie(n) und Leitungsmitteln zwischen Bauchdecke und innere Organe eingeschoben wird. Bei einer derartigen Ausführungsform kommt die flüssigkeitsdurchlässige Schicht folglich nur auf einem zentralen Anteil des Verbundes aus erster Folie und Leitungsmitteln oder des Verbundes aus erster Folie, Leitungsmitteln und zweiter Folie, zu liegen.

Falls die Leitungsmittel durch eine zentral angeordnete Leitungsplatte verbunden sind, kann es vorteilhaft sein, wenn die weitere flüssigkeitsdurchlässige Schicht eine entsprechende Aussparung zur Aufnahme der Leitungsplatte aufweist. Die Aussparung kann dabei lediglich als Vertiefung oder alternativ als durchgehende Öffnung ausgebildet sein.

Im Zusammenhang mit der vorliegenden Erfindung können auch mehr als eine flüssigkeitsdurchlässige Schichte auf der im Gebrauch von der Wunde abgewandten Seite des Verbundes aus erster Folie und Leitungsmitteln oder des Verbundes aus erster Folie, Leitungsmitteln und zweiter Folie vorgesehen sein, insbesondere mehr als eine Schicht eines porösen Polymerschaumstoffs. Die mehreren Schichten können dabei unterschiedliche Abmessungen aufweisen und in unterschiedlichen Dicken vorliegen.

Der erfindungsgemäßen Wundauflage kann weiterhin ein luftundurchlässiges Abdeckmaterial zum luftdichten Verschließen der Wunde und der Wundumgebung umfassen, wobei das Abdeckmaterial vorzugsweise einen Kleberand zum Befestigen des Abdeckmaterials auf der die Wunde umgebenden intakten Haut, aufweist. Unter "luftdichten Verschließen" soll hier nicht verstanden werden, dass keinerlei Gasaustausch zwischen dem Wundraum und seiner Umgebung stattfindet. Vielmehr ist mit "luftdichten Verschließen" in diesem Zusammenhang gemeint, dass unter Berücksichtigung der eingesetzten Unterdruckpumpe der für die Unterdrucktherapie von Wunden nötige Unterdruck aufrechterhalten werden kann. Es können deshalb auch Abdeckmaterialien eingesetzt werden, welche eine geringfügige Gas-Permeabilität aufweisen, so lange der für die Unterdrucktherapie notwendige Unterdruck aufrechterhalten werden kann.

In einer bevorzugten Ausführungsform der Erfindung umfasst das Abdeckmaterial zum luftdichten Verschließen der Wunde ein wasserunlösliches Polymer, oder eine Metallfolie. In einer besonders bevorzugten Ausführungsform der Erfindung handelt es sich bei dem wasserunlöslichen Polymer um Polyurethan, Polyester, Polypropylen, Polyethylen, Polyamid oder Polyvinylchlorid, Polyorganosiloxan (Silikon), oder um eine Mischung daraus.
Als Abdeckmaterial können auch Fertigprodukte eingesetzt werden, welche die vorstehend genannten Eigenschaften aufweisen.

Als besonders geeignetes Abdeckmaterial für die erfindungsgemäße Vorrichtung hat sich Polyurethanfilm der Marke Hydrofilm^{®} (Paul Hartmann AG, Deutschland) oder Visulin^{®} (Paul Hartmann AG, Deutschland) erwiesen.

Das Abdeckmaterial wird in der Wundumgebung oder am Wundrand so befestigt, dass ein luftdichter Wundverschluss gewährleistet ist. Dabei kann es zweckmäßig sein, wenn das Abdeckmaterial vollflächig selbstklebend ausgestattet ist oder einen selbstklebenden Rand aufweist. Alternativ können Befestigung und Abdichtung beispielsweise mit einer Klebefolie, mit einem flüssigen Kleber oder mit einer Abdichtmasse erfolgen.

In einer bevorzugten Ausführungsform der Erfindung umfasst das Abdeckmaterial eine Folie aus einem oder mehreren wasserunlöslichen Polymeren, wobei die Folie vollflächig selbstklebend ausgestattet ist oder einen selbstklebenden Rand aufweist.

Im Zusammenhang mit der erfindungsgemäßen Wundauflage wird des Weiteren ein Unterdruck-Anschlussstück zur funktionellen Verbindung des Wundraums mit einer außerhalb der Wundauflage befindlichen Unterdruckquelle beansprucht, wobei das Unterdruck-Anschlussstück derart gestaltet ist, dass ein Unterdruck im Wundraum herstellbar ist und Flüssigkeiten aus dem Wundraum absaugbar sind. Das Unterdruck-Anschlussstück wird bei der Verwendung der Wundauflage in der Unterdrucktherapie von Wunden vorzugsweise auf die von der Wunde abgewandte Seite des luftundurchlässigen Abdeckmaterials angebracht, wobei das Abdeckmaterial geeignete Öffnungen aufweist. Unterdruck-Anschlussstücke sind dem Fachmann auch unter der Bezeichnung "Port" bekannt. Das Unterdruck-Anschlussstück umfasst üblicherweise eine Verbindungsleitung, und einen Unterdruckadapter, um mit den weiteren Komponenten des Unterdrucksystems verbindbar zu sein.

Gemäß einer weiteren bevorzugten Ausführungsform werden Abdeckmaterial und das Mittel zur funktionellen Verbindung des Wundraums mit einer außerhalb des Abdeckmaterials befindlichen Unterdruckquelle bereits gebrauchsfertig miteinander verbunden zur Verfügung gestellt. Es ist ganz besonders bevorzugt, dass diese Ausführungsform eine Folie aus einem oder mehreren wasserunlöslichen Polymeren enthält, welche einen selbstklebenden Rand aufweist, da diese Anordnung das Anlegen des Verbandes wesentlich erleichtert.

Für die in der vorliegenden Erfindung beschriebene Wundauflage sind die in den deutschen Patentanmeldungen DE102010006272.3, DE 10 2010 006273.1 und DE 10 2009 060 596.7 offenbarten Unterdruck-Anschlussstücke besonders geeignet.

Gemäß einer alternativen Ausführungsform kann die funktionelle Verbindung des Wundraums mit einer außerhalb des Abdeckmaterials befindlichen Unterdruckquelle mit mindestens einer Verbindungsleitung hergestellt werden. Die mindestens eine Verbindungsleitung kann durch das Abdeckmaterial hindurchgeführt werden. Alternativ kann die mindestens eine Verbindungsleitung unter dem Rand des Abdeckmaterials durchgeführt werden. In beiden Fällen ist die Durchtrittsstelle luftdicht abzudichten, so dass der gewünschte Unterdruck im Verband aufrechterhalten werden kann. Als Abdichtmittel eignet sich beispielsweise eine Klebefolie, eine Klebemasse, oder ein Klebestreifen.

Bei der Verbindungsleitung kann es sich beispielsweise um einen Schlauch, um ein Rohr oder um einen anderen Körper mit einem Hohlraum handeln. Ein geeigneter Schlauch ist beispielsweise ein Silikon-Drainageschlauch.

Die erfindungsgemäße Wundauflage kann weiterhin ein Mittel umfassen, so dass der in der Vorrichtung tatsächlich vorhandene Unterdruck überprüfbar und gegebenenfalls einstellbar ist. Das Mittel kann sich im Wundraum oder an einer anderen geeigneten Stelle befinden. Alternativ ist es auch möglich einen Drucksensor in der Unterdruckleitung zwischen Wundverband und der Unterdruckquelle anzubringen.

Es ist vorgesehen, dass die vorgenannten Komponenten den die Wundbehandlung durchführenden Ärzten und Fachkräften als gebrauchsfertiges Set ("Kit") zur Verfügung gestellt werden. Die Erfindung bezieht sich daher auch ein gebrauchsfertiges Set zur Unterdruck-Wundbehandlung umfassend
a) eine Wundauflage nach einem der Ansprüche 1 bis 13,
b) mindestens eine flüssigkeitsdurchlässige Schicht zum Aufbringen auf die im Gebrauch von der Wunde abgewandte Seite der Wundauflage, wobei die mindestens eine flüssigkeitsdurchlässige Schicht bevorzugt eine oder mehrere flächenförmige Polster aus einem porösen Polymerschaumstoff, insbesondere aus PU, PVA oder Silikon umfasst,
c) ein luftundurchlässiges Abdeckmaterial zum luftdichten Verschließen der Wunde und der Wundumgebung, wobei das Abdeckmaterial vorzugsweise einen Kleberand aufweist,
d) ein Unterdruck-Anschlussstück zur funktionellen Verbindung des Wundraums mit einer außerhalb des Abdeckmaterials befindlichen Unterdruckquelle, so dass ein Unterdruck im Wundraum herstellbar ist und Flüssigkeiten aus dem Wundraum absaugbar sind, wobei das Unterdruck-Anschlussstück vorzugsweise zum Anbringen auf die im Gebrauch von der Wunde abgewandte Außenseite des Abdeckmaterials vorgesehen ist
   und wobei alle Komponenten a) bis d) jeweils einzeln steriler verpackt vorliegen.

Bei einer besonders bevorzugten Ausführungsform des Sets ist die Komponente a) insbesondere als Verbund aus erster Folie, Leitungsmitteln, Leitungsplatte und zweiter Folie ausgebildet, da ein solcher Verbund, wie bereits näher ausgeführt, besonders günstige Eigenschaften hinsichtlich der Fluiddurchlässigkeit beim Gebrauch aufweist und sich beim Verbandwechsel ohne Verklebung von den freigelegten inneren Organen oder von dem Omentum majus lösen lässt. Darüber hinaus ist es wünschenswert, dass bei der Wundauflage Taschen gemäß Anspruch 13 vorgesehen sind.

Für das gebrauchsfertige Set besonders geeignete Unterdruck-Anschlussstücke sind in den deutschen Patentanmeldungen Anmeldenummer DE102010006272.3, DE 10 2010 006273.1 und DE 10 2009 060 596.7 beschrieben.

Weiterhin kann das Set optionale Bestandteile wie beispielsweise eine oder mehrere zusätzliche flächenförmige Elemente einer flüssigkeitsdurchlässigen Schicht, Klebemittel zum Fixieren des Verbands, Abdichtmittel zur Herstellung einer luftundurchlässigen Abdichtung des Verbandes, Drucksensoren, Verbindungselemente für Drucksensoren, Schläuche, Verbindungsstücke für Schläuche, Desinfektionsmittel, Hautpflegemittel, pharmazeutische Zubereitungen oder eine Gebrauchsanweisung enthalten.

Vorzugsweise umfasst das Set weiterhin eine gebrauchsfertige Unterdruckeinheit. Die Unterdruckeinheit kann Komponenten wie beispielsweise eine Pumpe, ein oder mehrere Flüssigkeitsbehälter, eine Steuereinheit, eine Stromversorgung, elektrische Anschlussmitteln und Schläuche enthalten. Die Unterdruckeinheit kann auch eine Vorrichtung zur funktionellen Verbindung des Unterdruckverbandes mit einer vorhandenen stationären Unterdruckquelle enthalten.

Für das gebrauchsfertige Set besonders geeignete Unterdruck-Einheiten sind in den deutschen Patentanmeldungen Anmeldenummer DE102009038130.9 und DE102009038131.7 beschrieben. Ein für das Set besonders geeignete Unterdruck-Einheit ist unter der Bezeichnung VivanoTec (Hersteller Paul Hartmann AG, Heidenheim, Deuschland) kommerziell erhältlich.

Vorzugsweise werden alle Komponenten, bei denen dies aus medizinischer Sicht notwendig ist, steril abgepackt zur Verfügung gestellt. Der Vorteil des gebrauchsfertigen Sets besteht darin, dass der Unterdruckverband schnell, standardisiert und unkompliziert angelegt werden kann. Ein weiterer Vorteil besteht darin, dass alle im Wundbereich eingesetzten Bestandteile des Sets bereits sterilisiert zur Verfügung gestellt werden können. **Figurenlegende**
- 1.: Erste flexible Folie
- 2.: Leitungsmittel
- 3.: Hohlraum (Lumen) in Leitungsmittel
- 4.: Zweite flexible Folie
- 5.: Öffnung in erster flexibler Folie, welche mit einer Öffnung im Leitungsmittel in Deckung steht
- 6.: Öffnung in erster flexibler Folie, welche nicht mit einer Öffnung im Leitungsmittel in Deckung steht
- 7.: Haft- bzw. Befestigungspunkt zwischen erster und zweiter Folie
- 8.: Leitungsplatte
- 9.: Öffnung in Leitungsplatte
- 10.: Zentrale Öffnung in zweiter Folie
- 11.: Flüssigkeitsdurchlässige Schicht
- 12.: Zwischenraum zwischen Wundgrund (beispielweise Omentum majus oder freigelegte innere Organe) und Bauchdecke
- 13.: Wundgrund (beispielsweise Omentum majus oder freigelegte innere Organe)
- 14.: Bauchdecke
- 15.: Wundrand
- 16.: Luftundurchlässiger Abdeckfilm
- 17.: Öffnung in Abdeckfilm
- 18.: Unterdruck-Anschlussmittel (Port)
- 19.: Unterdruckleitung
- 20.: Kanister für Wundexsudat
- 21.: Unterdruckquelle
- 22.: Öffnung in der Wand des Leitungsmittels
- 23.: Endständige Öffnung in Leitungsmittel
- 24.: Hohlraum in Leitungsplatte
- 25.: Zur Mitte der Wundauflage hin offene Tasche
- 26.: Naht bzw. Verklebungsbereich von Tasche mit erster oder zweiter flexibler Folie
- 27.: Zur Mitte der Wundauflage hin weisende Öffnung der Tasche
- 28.: Lumen der Unterdruckleitung

### Figuren

Nachstehend wird die erfindungsgemäße Vorrichtung zur Unterdrucktherapie von Wunden anhand schematischer Zeichnungen näher erläutert. Die Erfindung soll jedoch nicht auf die in den Zeichnungen oder in der Beschreibung der Zeichnung dargestellten Ausgestaltungen reduziert verstanden werden. Vielmehr umfasst die erfindungsgemäße Vorrichtung auch Kombinationen der Einzelmerkmale der alternativen Formen.
Fig. 1 zeigt eine Ausführungsform der erfindungsgemäßen Wundauflage zur Verwendung bei der Unterdrucktherapie in der Aufsicht auf die wundabgewandte Seite der Wundauflage.
Fig. 2 zeigt einen Querschnitt der in Fig. 1 dargestellten Wundauflage entlang der Linie A-A.
Fig. 3 zeigt eine weitere Ausführungsform der erfindungsgemäßen Wundauflage in der Aufsicht auf die wundabgewandte Seite der Wundauflage.
Fig. 4 zeigt die Ausführungsform gemäß Fig. 3 im Querschnitt entlang der Linie B-B.
Fig. 5 zeigt die Ausführungsform gemäß Fig. 3 im Querschnitt entlang der Linie C-C.
Fig. 6 zeigt die Ausführungsform gemäß Fig. 3 im Querschnitt entlang der Linie D-D.
Fig. 7 zeigt in Darstellung eine besonders bevorzugte Ausführungsform eines an eine Wunde angelegten Unterdruckverbandes im Querschnitt.

### Ausführliche Beschreibung der Erfindung

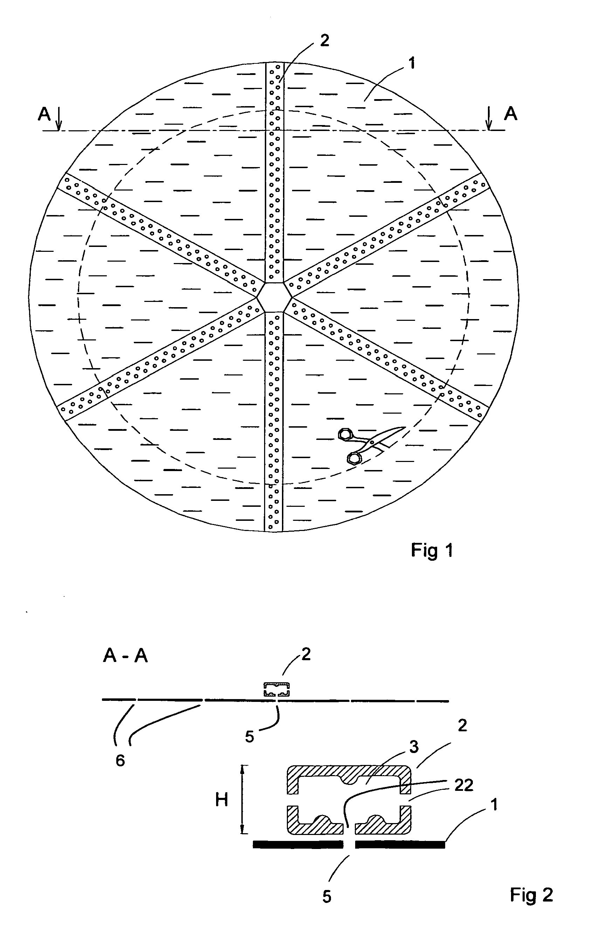

Bei der in Fig. 1 und Fig. 2 dargestellten Ausführungsform sind mehrere Abschnitte eines Leitungsmittels (2) aus einem biegsamen elastomeren Material mit einer Dickenerstreckung (H) von höchstens 30 mm sternförmig auf einer ersten flexiblen Folie (1) angeordnet. Das Leitungsmittel (2) weist eine Vielzahl von seitlichen Öffnungen (22) und mindestens eine endständige Öffnung (23) auf. Dabei ist es vorteilhaft, wenn die Leitungsmittel (2) mit der ersten Folie (1) unlösbar verbunden werden, beispielsweise durch Verkleben. Das Leitungsmittel (2) soll dabei flach ausgebildet sein, so dass es mit mindestens 70% seiner senkrecht auf die erste Folie (1) projizierten Fläche unlösbar und flächenhaft verbunden werden kann. Unter flach wird in diesem Zusammenhang verstanden, dass das Verhältnis Breite zu Höhe (H) des Leitungsmittels mindestens 1,25, insbesondere mindestens 1,50 betragen soll. Das Leitungsmittel (2) sollte möglichst aus einem elastomeren Material gefertigt sein, welches eine Shore A Härte von höchstens 60, vorzugsweise von weniger als 50, besonders bevorzugt von weniger als 45, insbesondere von weniger als 40 aufweist. Wie durch das Scherensymbol in Fig. 1 dargestellt, kann die Wundauflage durch Zuschneiden auf die für die Behandlung der Wunde benötigte Größe angepasst werden, wobei sowohl erste Folie (1) als auch Leitungsmittel (2) durchtrennt werden. Gemäß Fig. 2 kann die erste Folie (1) sowohl Öffnungen (6) in den nicht mit dem Leitungsmittel (2) in Kontakt tretenden Bereichen, als auch Öffnungen (5), welche mit den Öffnungen (22) im Leitungsmittel (2) in Deckung stehen, aufweisen. Wie in Fig. 2 dargestellt (vergrößerter Ausschnitt) ermöglichen zueinander in Deckung stehende Öffnungen (5) von erster Folie (1) und Leitungsmittel (2) eine direkte Fluid-Kommunikation vom Wundraum und Lumen (3) des Leitungsmittels (2). Um derartige, zueinander in Deckung stehende Öffnungen (5) durch erste Folie (1) und Leitungsmittel (2) zu erhalten, ist es am einfachsten, wenn die Öffnungen erst nach dem Verkleben von Leitungsmittel und erster Folie eingebracht werden. Gemäß der in Fig. 2 (vergrößerter Ausschnitt) dargestellten besonders vorteilhaften Ausgestaltung der Erfindung weist das Leitungsmittel (2) sowohl seitlich angebrachte Öffnungen (22), als auch Öffnungen in der zur ersten Folie (1) zeigenden Wand auf. Jedoch ist die Erfindung nicht auf die in Fig. 2 gezeigte Anordnung der Öffnungen (22) im Leitungsmittel (2) beschränkt. Vielmehr können die Öffnungen (22) nur an einer, zwei, drei oder aber an allen Außenwänden des Leitungsmittels (2) vorhanden sein. Gemäß der in Fig. 2 dargestellten Ausführungsform weist das Leitungsmittel weiterhin in seinem Querschnitt einen weitestgehend rechteckigen äußeren Umfang auf. Es sind auch andere äußere Formen denkbar, solange das Leitungsmittel (2) insgesamt flach ausgebildet ist. Das Leitungsmittel kann auf der zum Lumen (3) hin weisenden Innenseite Vorwölbungen oder Verdickungen aufweisen, die der Stabilisierung des Leitungsmittels (2) dienen.

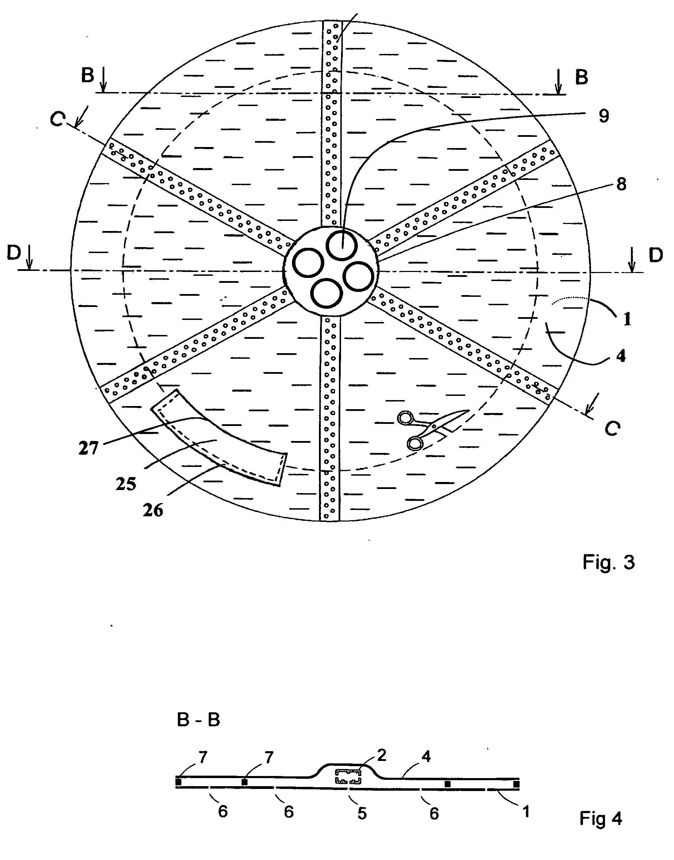

Eine weitere bevorzugte Ausführungsform ist in Fig. 3 (Aufsicht), sowie in Fig. 4, Fig. 5 und Fig. 6 (Querschnitte) dargestellt. Gemäß dieser Ausführungsform umfasst die Wundauflage eine zweite flexible Folie (4), welche auf dem Verbund aus erster flexibler Folie (1), Leitungsmittel (2) und Leitungsplatte (8) aufgebracht ist. Die zweite flexible Folie (4) ist in der dargestellten Ausführungsform eine transparente Folie (in der Aufsicht der Fig. 3 sind die weiteren, unterhalb der zweiten Folie angeordneten Komponenten daher so dargestellt, als wäre die zweite Folie nicht vorhanden). Die Leitungsmittel (2) sind sternförmig angeordnet und im Zentrum durch eine Leitungsplatte (8) flüssigkeitsleitend verbunden. Die Leitungsplatte (8) ist in Form einer flachen Dose mit einem Hohlraum (24) ausgestaltet und kann dabei mit der ersten flexiblen Folie (1) und/oder der zweiten flexiblen Folie (4) unlösbar verbunden sein, beispielsweise durch Verkleben. Gemäß einer besonders vorteilhaften Ausbildung der Erfindung ist die zweite flexible Folie (4) weitestgehend fluidundurchlässig ausgebildet und weist lediglich eine einzige, vorzugsweise im zentralen Bereich angeordnete, Öffnung (10) auf. Eine unlösbare Verbindung der zweiten flexiblen Folie (4) mit der Leitungsplatte (8) ist bei dieser Ausführungsform besonders vorteilhaft.

Wundfluid kann aus der Wunde über Öffnungen (5, 22) in der ersten Folie (1) und im Leitungsmittel (2) zunächst in das Lumen (3) des Leitungsmittels (2) gelangen. Zusätzlich kann Wundfluid zunächst über Öffnungen (6) in der ersten flexiblen Folie (1) in den Zwischenraum zwischen erster (1) und zweiter Folie (4) gelangen und dann über seitlich am Leitungsmittel angebrachte Öffnungen (22) in das Lumen (3) des Leitungsmittels (2) gelangen. Vom Lumen (3) des Leitungsmittels (2) kann Wundfluid weiterhin in das Lumen (24) der Leitungsplatte (8) gesaugt werden. Das Lumen (24) der Leitungsplatte (8) steht mit einer Unterdruckverbindung über wundabseitig lokalisierte Öffnungen (9) in der Leitungsplatte (8) in Fluid-Verbindung. Es ist besonders vorteilhaft, wenn erste (1) und zweite Folie (4) über Haftpunkte (7) miteinander unlösbar verbunden werden. Bei einer derartigen Ausgestaltung kann weiterhin auf eine Verklebung des oder der Leitungsmittel (2) mit erster (1) und/oder zweiter Folie (4) verzichtet werden, da diese von den beiden Folien taschenartig gehalten werden. Es ist jedoch insbesondere bei dieser Ausführungsform sinnvoll, die zweite flexible Folie (4) mit dem Randbereich der Leitungsplatte (8) zu verkleben. Durch die punktartige Verbindung von erster (1) und zweiter (4) Folie entsteht ein Zwischenraum, der Fluidfluss ermöglicht. Hierbei ist es wünschenswert, dass die Leitungsmittel (2) über seitliche Öffnungen (22) verfügen, so dass ein Fluidabfluss aus dem Zwischenraum in das Lumen (3) der Leitungsmittel (2) erfolgen kann.

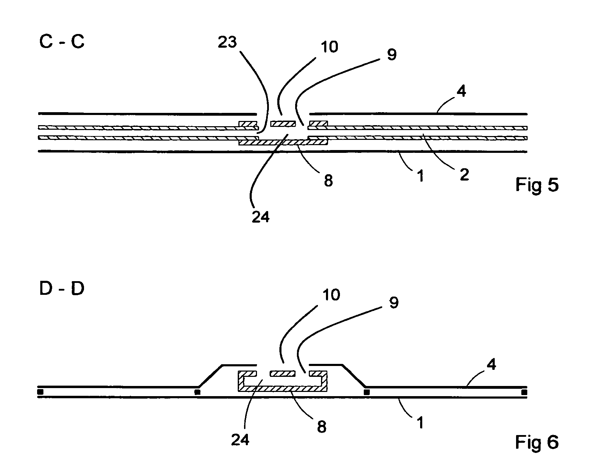

Die Leitungsmittel (2) sind mit der Leitungsplatte (8) Fluid-leitend verbunden (Fig. 5). Dies wird erreicht, indem ein an seinem Ende offenes Leitungsmittel (2) in eine Öffnung der Leitungsplatte (8) gesteckt oder auf andere Weise befestigt ist, so dass das Leitungsmittel (2) durch mindestens eine endständige Öffnung (23) mit einer Unterdruckquelle (21) Fluid-leitend verbunden ist. Die Leitungsplatte (8) kann mit der ersten flexiblen Folie (1) unlösbar verbunden sein, z. B. durch Verkleben, oder alternativ auf der ersten flexiblen Folie (1) lose aufliegen. Die Leitungsplatte (8) weist eine oder mehrere Öffnungen (9) nach oben, d. h. nach der im Gebrauch von der Wunde wegweisenden Seite hin auf. Wie aus Fig. 5 hervorgeht, befindet sich die Leitungsplatte (8) zwischen erster Folie (1) und zweiter Folie (4). Die zweite flexible Folie (4) weist dabei einen zentralen Ausschnitt (10) auf, so dass Fluidkommunikation zwischen dem Hohlraum (24) der Leitungsplatte (8) und einem Unterdruckanschlussmittel (Port) stattfinden kann. Zweckmäßigerweise ist, wie in Fig.5 und Fig. 6 dargestellt, die zentrale Öffnung in der zweiten flexiblen Folie (4) derart gestaltet, dass die inneren Ränder der Folie (4) auf den oberen (d.h. im Gebrauch von der Wunde weg weisenden) peripheren Bereichen der Leitungsplatte (8) zu liegen kommen und dort unlösbar miteinander verbunden sind. Dies kann beispielsweise durch Verkleben erfolgen. Die Verbindung der zweiten flexiblen Folie (4) mit der Leitungsplatte (8) kann, wie in Fig.5 und Fig. 6 dargestellt, insbesondere durch Verklebung der zweiten flexiblen Folie (4) mit dem peripheren Bereich der oberen (d.h. im Gebrauch von der Wunde weg weisenden) Seite der Leitungsplatte (8) erfolgen,

Fig. 3 zeigt des Weiteren eine zur Mitte der Wundauflage hin offene Tasche (25) mit einer Tiefe von höchstens 6 cm. In Fig. 3 ist nur eine Tasche beispielhaft gezeigt. Vorzugsweise sollten jedoch mehrere derartige Taschen, möglichst in gleichmäßigem Abstand um den Umfang der Wundauflage verteilt, aufgebracht werden. Insbesondere sind die eine oder mehreren Taschen auf mindestens 50 % des Umfangs der Wundauflage vorgesehen. Derartige Taschen (25) erleichtern das das gleichmäßige Aufbringen und Auslegen der Wundauflage auf den Wundgrund (13). Insbesondere erleichtern die Taschen das Einschieben der Wundauflage zwischen Wundgrund (13) und Bauchdecke (14), beispielsweise mittels eines Bauchspatels. Die Taschen (25) können durch Einfalten des Randes der ersten flexiblen Folie (1) oder des Verbundes aus erster (1) und zweiter Folie (4) und/oder durch das Aufbringen weiterer Folienstücke gebildet werden. Bei dem in Fig. 3 dargestellten Ausführungsbeispiel ist die Tasche (25) durch das Aufbringen eines weiteren Folienstücks gebildet. Das Folienstück ist durch eine Naht oder Verklebung (26) an der zweiten flexiblen Folie (4) befestigt und weist eine zur Mitte der Wundauflage hin weisende Öffnung (27) auf. Es ist auch möglich mehrere konzentrisch aufgebrachte Reihen von Taschen anzubringen. Dies hat den Vorteil, dass beim Zuschneiden des Randes der Wundauflage (insofern dabei Taschen teilweise oder vollständig abgeschnitten werden) zusätzliche, weiter innen angebrachte intakte Taschen zur Verfügung stehen, welche ein Einbringen der Wundauflage zwischen Wundgrund (13) und Bauchdecke erleichtern.

Weiterhin kann auf der im Gebrauch von der Wunde wegweisenden Seite der zweiten Folie eine oder mehrere flüssigkeitsdurchlässige Schichten aufgebracht werden. Ein besonders geeignetes Material für eine derartige flüssigkeitsdurchlässige Schicht umfasst einen porösen Polymerschaum, insbesondere einen offenzelligen porösen Polymerschaum. Bei der Verwendung einer flüssigkeitsdurchlässigen Schicht, welche über der zweiten flexiblen Folie (4) angeordnet ist, kann Fluidkommunkation vom Hohlraum (24) der Leitungsplatte (8) über die flüssigkeitsdurchlässige Schicht zu einem Unterdruckanschlussmittel (Port) erfolgen.

Fig. 6 zeigt einen Schnitt entlang der Linie D-D von Fig. 3. Im Zentrum der Wundauflage befindet sich die Leitungsplatte (8) mit nach oben hinweisenden Öffnungen (9). Die Ränder der zweiten flexiblen Folie (4) liegen dem Rand der Leitungsplatte (8) auf und sind vorzugsweise mit dem Rand unlösbar verbunden, beispielsweise durch Verkleben. In den peripheren Bereichen außerhalb der Leitungsplatte (8) stehen erste flexible Folie (1) und zweite flexible Folie (4) in direkten Kontakt zueinander. Vorzugsweise sind erste (1) und zweite (4) Folie über Haftpunkte (7) verbunden. Die Haftpunkte (7) können zu einer geringfügigen Beabstandung zwischen erster (1) und zweiter (4) Folie führen. Bei der in Fig. 6 dargestellten Ausführungsform weist die erste flexible Folie (1) eine Vielzahl von über die Fläche der ersten Folie verteilten Öffnungen auf (5, 6), während die zweite Folie (4) lediglich über eine einzige zentrale Öffnung (10) verfügt. Bei dieser Anordnung kann optional eine flüssigkeitsdurchlässige Schicht auf die von der Wunde weg weisende Seite der zweiten Folie (4) aufgebracht werden, ohne dass die flüssigkeitsdurchlässige Schicht mit dem Wundgrund in Kontakt kommt. Dies ist vorteilhaft, da auf diese Weise eine Verklebung der flüssigkeitsdurchlässigen Schicht mit dem Wundgrund vermieden wird.

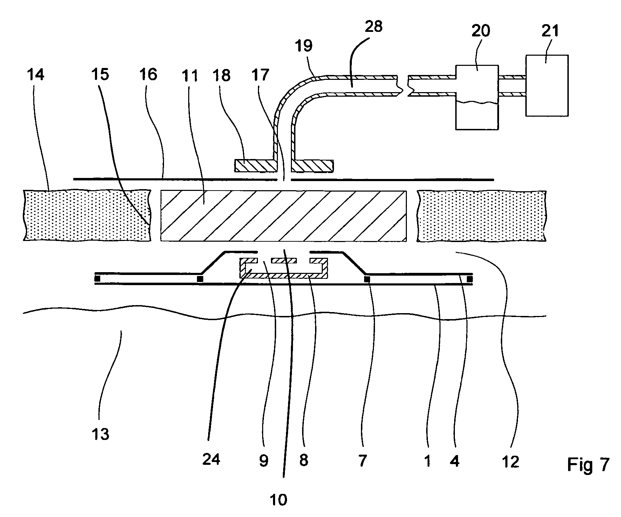

Fig. 7 zeigt in schematischer Darstellung die Anwendung einer bevorzugten Ausführungsform der erfindungsgemäßen Wundauflage bei der Behandlung einer Abdominalwunde. Die Wundauflage wird auf den Wundgrund (13), beispielsweise auf das Omentum majus oder freigelegte innere Organe, aufgebracht. Der periphere Bereich der Wundauflage befindet sich im Zwischenraum (12) zwischen Wundgrund (13) und Bauchdecke (14). Die Wundauflage umfasst gemäß der in Fig. 7 gezeigten Ausführungsform eine flüssigkeitsdurchlässige Schicht (11), die vorzugsweise einen porösen Polymerschaum umfasst. Die flüssigkeitsdurchlässige Schicht (11) soll hinsichtlich ihrer Größe so beschaffen sein, dass sie mit den Wundrändern (15) in Kontakt tritt. Dies ist insbesondere bei der Verwendung eines porösen Polymerschaums als flüssigkeitsdurchlässige Schicht (11) vorteilhaft, da bekannt ist, dass der Kontakt des Wundrandes (15) mit einem porösen Polymerschaum das Wachstum des Wundrandgewebes fördert. Die Wunde und die Wundumgebung werden mit einem luftundurchlässigen Abdeckfilm (16) überdeckt. Der Abdeckfilm (16) weist vorzugsweise auf der zur Wunde hinzeigenden Seite teilweise oder vollflächig eine Klebeschicht (nicht dargestellt) auf, so dass er auf dem die Wunde umgebenden Hautbereich festgeklebt werden kann. Weiterhin wird im zentralen Bereich des Abdeckfilms (16) mindestens eine Öffnung (17) eingebracht, um Fluidtransport zu ermöglichen. Über den Bereich der Öffnung (17) wird ein Unterdruckanschlussmittel (18) befestigt. Das Unterdruckanschlussmittel (18) ist über die Unterdruckleitung (19) mit einem Kanister (20) und einer Unterdruckquelle (21) verbunden.

Im Zusammenhang mit der vorliegenden Erfindung besonders geeignete tragbare Unterdruck-Einheiten, welche Kanister (20) und Unterdruckquelle (21) trennbar in einer einzigen Einheit umfassen, sind in den deutschen Patentanmeldungen Anmeldenummer DE102009038130.9 und DE102009038131.7 beschrieben. Hinsichtlich geeigneter Unterdruckanschlussmittel (18) wird auf die bereits erwähnten deutschen Patentanmeldungen Anmeldenummer DE 10 2010 003 273.1, DE 10 2010 003 272.3 und DE 10 2009 060 596.7 verwiesen.

### Herstellung der Wundauflage

Die Erfindung betrifft weiterhin ein Verfahren zur Herstellung einer Wundauflage für die Unterdrucktherapie, wobei die Wundauflage insbesondere zur Behandlung abdominaler Wunden vorgesehen ist, umfassend die Schritte
a) Bereitstellen einer ersten flexiblen Folie (1) mit einer ersten und einer zweiten Seite, wobei die erste Seite zum Aufbringen auf freigelegte innere Organe oder auf das Omentum majus, vorgesehen ist und wobei die Folie eine Vielzahl von über die Fläche verteilten Öffnungen (5, 6) aufweist,
b) Bereitstellen von mindestens drei Leitungsmitteln (2) aus einem biegsamen elastomeren Material mit einer Dickenerstreckung (H) von höchstens 30 mm, wobei die Leitungsmittel mindestens einen durchgehenden Hohlraum (3) aufweisen, und wobei die Leitungsmittel (2) weiterhin eine Vielzahl von Öffnungen aufweisen,
c) Aufbringen der Leitungsmittel (2) auf die zweite Seite der ersten Folie (1). Das Verfahren kann gemäß einer bevorzugten Ausführungsform den weiteren Schritt
d) Aufbringen einer zweiten Folie (4) auf den Verbund aus erster Folie (1) und Leitungsmitteln (2) umfassen.

Gemäß einer weiteren bevorzugten Ausführungsform ist das Leitungsmittel (2) aus Schritt b) aus einem Material gefertigt ist, welches eine Shore A Härte von höchstens 60, vorzugsweise von weniger als 50, insbesondere von weniger als 45, besonders bevorzugt von weniger als 40 aufweist.

Die Erfindung betrifft des Weiteren gemäß einer besonders bevorzugten Ausführungsform ein Verfahren zur Herstellung einer Wundauflage, welche einen Verbund aus mindestens drei sternförmig angeordneten Leitungsmitteln (2) und einer Leitungsplatte (8) umfasst. In der Praxis erweist es sich dabei als vorteilhaft, zunächst den Verbund aus den mindestens drei Leitungsmitteln (2) und einer Leitungsplatte (8) herzustellen, und anschließend den Verbund auf die erste flexible Folie (1) aufzubringen. Das Verfahren gemäß dieser besonders bevorzugten Ausführungsform umfasst die Schritte
a) Bereitstellen einer ersten flexible Folie (1) mit einer ersten und einer zweiten Seite, wobei die erste Seite zum Aufbringen auf freigelegte innere Organe oder auf das Omentum majus, vorgesehen ist und wobei die Folie (1) eine Vielzahl von über die Fläche verteilten Öffnungen (5, 6) aufweist,
b) Bereitstellen eines Verbundes aus mindestens drei Leitungsmitteln (2) mit einer Leitungsplatte (8), wobei die Leitungsmittel (2) ein biegsames elastomeres Material mit einer Dickenerstreckung (H) von höchstens 30 mm umfassen, und wobei die Leitungsmittel (2) weiterhin mindestens einen durchgehenden Hohlraum (3) und eine Vielzahl von Öffnungen (22) aufweisen,
c) Aufbringen des Verbundes aus Leitungsmitteln (2) und Leitungsplatte (8) auf die zweite Seite der ersten Folie (1).

Das Verfahren kann gemäß einer weiteren besonders bevorzugten Ausführungsform den weiteren Schritt
d) Aufbringen einer zweiten Folie (4) auf den Verbund aus erster Folie (1), Leitungsmitteln (2) und Leitungsplatte (8) umfassen, wobei die zweite Folie auf die im Gebrauch von der Wunde weg weisenden Seite des Verbundes aufgebracht wird.

Gemäß einer weiteren, für die praktische Anwendung der Wundauflage in der Unterdrucktherapie besonders vorteilhaften Ausführungsform ist das Leitungsmittel (2) aus Schritt b) aus einem Material gefertigt ist, welches eine Shore A Härte von höchstens 60,
vorzugsweise von weniger als 50, insbesondere von weniger als 45, besonders bevorzugt von weniger als 40 aufweist.

### Anwendung

Bei der Anwendung zur Unterdrucktherapie großflächiger Wunden im Abdominalbereich wird zunächst die erfindungsgemäße Wundauflage auf den Wundgrund (13) gelegt. Die Randbereiche der Wundauflage können dann über eine Tiefe von ca. 1 bis 15 cm zwischen Bauchdecke (14) und Wundgrund (13) eingeschoben werden, um eine flächige Abdeckung des Wundgrundes (13) zu erreichen. Optional vorgesehene zur Mitte der Wundauflage hin offene Taschen (25) können das Einschieben des Randbereichs der Wundauflage zwischen Bauchdecke und Wundgrund erleichtern, indem der behandelnde Arzt einen Bauch- oder Darmspatel in die Taschen einführt und sodann den Randbereich der Wundauflage zwischen Bauchdecke und den Wundgrund einbringt.
Vorteilhafterweise umfasst die Wundauflage eine oder mehrere flüssigkeitsdurchlässige Schichten (11). Die eine oder mehreren flüssigkeitsdurchlässigen Schichten können einen porösen Polymerschaumstoff umfassen. Dabei ist es für die Wundheilung sehr förderlich, wenn die flüssigkeitsdurchlässige Schicht (11) dergestalt an die Form der Wunde angepasst wird, dass die Wundränder (15) in einem vollständigen Kontakt mit der einen oder mehreren flüssigkeitsdurchlässigen Schicht (11) stehen.
Zum luftdichten Verschließen des Wundbereichs wird ein luftundurchlässiges Abdeckmaterial (16) über die Wunde gelegt. Die Ränder des Abdeckmaterials (16) werden auf die intakte Haut geklebt. Des Weiteren wird ein Unterschlussanschlussstück (18) angebracht, um eine funktionelle Verbindung des Wundraums mit einer außerhalb des Abdeckmaterials (16) befindlichen Unterdruckquelle (21), beispielsweise einer Unterdruckpumpe, herzustellen, so dass ein Unterdruck im Wundraum herstellbar ist und Flüssigkeiten aus dem Wundraum absaugbar sind. Das Unterdruckanschlussstück (18) wird vorzugsweise auf die von der Wunde abgewandte Außenseite des Abdeckmaterials (16) aufgeklebt, wobei vor dem Aufkleben in das ansonsten luftundurchlässige Abdeckmaterial (16) eine geeignete Öffnung (17) geschnitten wird. Die Unterdrucktherapie wird eingeleitet, indem das Unterdruckanschlussstück (18) mit einer Unterdruckquelle (21) verbunden wird und ein konstanter Unterdruck oder ein zeitlich variierenden Unterdruck für eine Dauer von einigen Minuten bis zu mehreren Tagen angelegt wird.
Unter "konstantem Unterdruck" wird hier verstanden, dass der Unterdruck während der Behandlung im Wesentlichen konstant gehalten wird. "Im Wesentlichen konstant" bedeutet, dass während der Behandlung geringfügige Veränderungen des Unterdrucks, beispielsweise um 15 % nach oben oder unten, auftreten können.
Ein bevorzugter konstanter Unterdruck ist der Bereich von mindestens 80 mm Hg bis höchstens 250 mm Hg, vorzugsweise 125 mm Hg.
Unter "zeitlich variierendem Unterdruck" wird hier verstanden, dass der Unterdruck während der Behandlung gezielt variiert wird. Unter der gezielten Variation des Luftdrucks werden diejenigen Variationen des Luftdrucks verstanden, die einsetzen, wenn nach Anlegen des Unterdruckverbandes ein erster Sollwert für den Unterdruck erreicht wurde. Dagegen ist die erste Anstiegsphase des Unterdrucks, die nach dem Anlegen des Verbandes bis zum Erreichen des ersten Sollwertes auftritt, nicht von dem Begriff "zeitlich variierender Unterdruck" umfasst. Dies gilt analog für die am Ende der Behandlung nötige Absenkung des Luftdrucks auf den Umgebungsluftdruck, die gleichfalls nicht von dem Begriff "zeitlich variierender Unterdruck" umfasst ist.
Der "zeitlich variierende Unterdruck" ist durch den Umgebungsluftdruck nach unten und durch einen maximalen Unterdruck von 250 mm Hg, vorzugsweise 150 mm Hg, insbesondere 125 mm Hg, nach oben begrenzt. Der während der Behandlung applizierte tatsächliche Unterdruck bewegt sich innerhalb dieser durch ihre Eckwerte definierten Spanne. Der "zeitlich variierende Unterdruck" umfasst also beispielsweise einen ein- oder mehrmaligen Wechsel zwischen einem oder mehreren höheren Unterdruckwerten und einem oder mehreren geringeren Unterdruckwerten. Ebenso umfasst der "zeitlich variierende Unterdruck" einen während der Behandlung erfolgenden gezielten ein- oder mehrmaligen Wechsel zwischen dem Umgebungsdruck und einem oder mehreren höheren Unterdruckwerten.
Bei einer bevorzugten Ausführungsform beträgt der maximale Unterdruck für einen zeitlich variierenden Unterdruck 125 mm Hg. Die untere Grenze für die Variation des Unterdrucks stellt bei dieser Ausführungsform der Umgebungsluftdruck dar. Während der Behandlung variiert der Unterdruck zwischen oder innerhalb dieser Eckwerte.
Bei einer weiteren bevorzugten Ausführungsform beträgt der maximale Unterdruck für einen zeitlich variierenden Unterdruck 125 mm Hg. Die untere Grenze für die Variation des
Unterdrucks beträgt bei dieser Ausführungsform 20 mm Hg. Während der Behandlung variiert der Unterdruck zwischen oder innerhalb dieser Eckwerte.
Bei beiden vorgenannten Ausführungsformen kann der Wechsel zwischen dem oberen und dem unterem Druckwert periodisch oder nicht-periodisch erfolgen. Ein periodischer Wechsel ist bevorzugt. Die Zeitintervalle, in denen der höhere Unterdruck und in denen der geringere Unterdruck oder Umgebungsdruck gehalten wird können jeweils unterschiedlich lang sein. Vorzugsweise wird ein geringerer Unterdruck länger gehalten als ein höherer Unterdruck. Geeignete Zeitintervalle, in denen jeweils eine bestimmte Unterdruckeinstellung oder der Umgebungsdruck gehalten wird sind beispielsweise 1 min, 2 min, 5 min, 10 min, 30 min, 1 h, 12h oder 24h.
Besonders bevorzugt ist ein variierender Unterdruck mit den nachstehend genannten Parametern, wobei während der Behandlung kontinuierlich in den angegebenen Zeitabständen zwischen den beiden Unterdruckwerten gewechselt wird:
Ein Unterdruck von 125 mm Hg während 2 min,
danach
ein Unterdruck von 20 mm Hg während 5 min.

Entsprechend wird mit der vorliegenden Erfindung ein Verfahren zur Unterdrucktherapie einer Wunde, insbesondere einer Wunde im Abdominalbereich, beschrieben, umfassend die Schritte
a) Auflegen eines Verbundes aus erster Folie und Leitungsmitteln oder eines Verbundes aus erster Folie, Leitungsmitteln und Leitungsplatte oder eines Verbundes aus erster Folie, Leitungsmitteln und zweiter Folie oder eines Verbundes aus erster Folie, Leitungsmitteln, Leitungsplatte und zweiter Folie auf die Wunde,
b) gegebenenfalls Aufbringen einer oder mehrerer flüssigkeitsdurchlässigen Schichten (11) auf die erste oder zweite Folie,
c) Abdichtung der Wunde unter Verwendung einer geeigneten luftdichten Abdeckung 16),
d) Anbringen eines Unterdruckanschlussmittels (18),
e) Anlegen von Unterdruck für mindestens 30 Minuten und für höchstens 5 Tage.

## Patentansprüche

1. Wundauflage zur Verwendung bei der Unterdrucktherapie abdominaler Wunden, umfassend
- eine erste flexible Folie (1) mit einer ersten und einer zweiten Seite, wobei die erste Seite zum Aufbringen auf freigelegte innere Organe oder auf das Omentum majus, vorgesehen ist,
- auf der zweiten Seite der ersten Folie (1) aufgebrachte Leitungsmittel (2) aus einem biegsamen elastomeren Material mit einer Dickenerstreckung (H) von höchstens 30 mm, wobei die Leitungsmittel (2) mindestens einen durchgehenden Hohlraum (3) aufweisen, wobei es sich bei den Leitungsmitteln (2) um Drainageschläuche für medizinische Zwecke handelt, wobei die Drainageschläuche PU, PVC, PE, PET, PTFE, Silikon oder eine Mischung daraus umfassen,
und wobei sowohl die Leitungsmittel (2) als auch die erste Folie (1) eine Vielzahl von Öffnungen aufweisen, so dass eine Fluid-Verbindung zwischen den Leitungsmitteln (2) und dem Wundraum herstellbar ist,
und wobei weiterhin die Leitungsmittel (2) durch mindestens eine weitere Öffnung, mit einer Unterdruckquelle (21) Fluid-leitend verbindbar ist,
und wobei die Leitungsmittel (2) flach ausgebildet sind und weiterhin
a) mit wenigstens 70 % ihrer senkrecht auf die erste Folie (1) projizierten Fläche mit der zweiten Seite der ersten Folie (1) unlösbar und flächenhaft verbunden sind oder
b) mit wenigstens 85 % ihrer senkrecht auf die erste Folie (1) projizierten Fläche auf der zweiten Seite der ersten (1) Folie aufliegen, wobei die Leitungsmittel (2) durch Befestigungsmittel auf der ersten Folie (1) gehalten werden,
**dadurch gekennzeichnet,**
**dass** mindestens drei Leitungsmittel (2) auf die zweite Seite der ersten Folie (1) aufgebracht sind und dass die Leitungsmittel (2) in einer sternförmigen Anordnung oder in einer parallelen Anordnung aufgebracht sind.

2. Wundauflage nach Anspruch 1, wobei die Leitungsmittel (2) aus einem Material gefertigt sind, welches eine Shore A Härte (gemessen nach DIN 53505 vom August 2000, bei einer Temperatur von 23°C) von höchstens 60 aufweist.

3. Wundauflage nach einem der vorangehenden Ansprüche, wobei es sich bei den Öffnungen um Löcher handelt und wobei die Summe der offenen Fläche der Löcher der ersten Folie (1) mindestens 0,1 % und höchstens 20 % der Flächenerstreckung der Folie beträgt.

4. Wundauflage nach einem der vorangehenden Ansprüche weiterhin umfassend eine zweite flexible Folie (4), wobei die zweite Folie (4) auf die im Gebrauch von der Wunde abgewandte Seite des Verbundes aus erster Folie (1) und Leitungsmitteln (2) aufgebracht ist.

5. Wundauflage nach Anspruch 4, wobei die zweite Folie (4) eine Vielzahl von über die Fläche verteilten Öffnungen aufweist, welche zum Durchleiten von Fluid geeignet sind.

6. Wundauflage nach Anspruch 4, wobei die zweite Folie weitestgehend Fluidundurchlässig ausgebildet ist und lediglich eine einzige im zentralen Bereich angeordnete Öffnung (10) aufweist.

7. Wundauflage nach einem der vorangehenden Ansprüche, wobei es sich bei der ersten flexiblen Folie (1) und/oder - falls vorhanden - bei der zweiten flexiblen Folie (4) um eine thermoplastische Folie für Medizinzwecke aus PU, PE, PET, PVC, Silikon oder einer Mischung daraus handelt.

8. Wundauflage nach einem der vorangehenden Ansprüche 4 bis 6, wobei die zweite flexible Folie (4)
a) zumindest stellenweise mit den mindestens drei Leitungsmitteln (2) unlösbar verbunden ist,
und / oder
b) zumindest stellenweise mit der ersten flexiblen Folie (1),
unlösbar verbunden ist.

9. Wundauflage nach einem der vorangehenden Ansprüche, weiterhin umfassend eine Leitungsplatte (8), welche auf die zweite Seite der ersten flexiblen Folie (1) aufgebracht ist und an der die Enden der mindestens drei Leitungsmittel (2) befestigt werden.

10. Wundauflage nach Anspruch 9, wobei die Leitungsplatte (8) in Form einer flachen Dose mit einem inneren Hohlraum (24) ausgebildet ist, und wobei weiterhin die Enden der mindestens drei Leitungsmittel (2) so eingebracht werden, dass eine Fluid-Verbindung zwischen den Lumina (3) der Leitungsmittel (2) und dem Hohlraum der Leitungsplatte (24) herstellbar ist.

11. Wundauflage nach einem der vorangehenden Ansprüche, weiterhin umfassend eine oder mehrere flüssigkeitsdurchlässige Schichten (11), zum Aufbringen auf die im Gebrauch von der Wunde abgewandte Seite der Wundauflage.

12. Wundauflage nach Anspruch 11, wobei die flüssigkeitsdurchlässige Schicht (11) einen porösen Polymerschaumstoff aus PU, PVA oder Silikon, umfasst.

13. Wundauflage nach einem der vorangehenden Ansprüche, wobei die Wundauflage auf der im Gebrauch von der Wunde abgewandte Seite mindestens eine oder mehrere randständige und zur Mitte der Wundauflage hin offene Taschen (25) mit einer Tiefe von höchstens 6 cm aufweist, welche das gleichmäßige Aufbringen und Auslegen der Wundauflage auf den Wundgrund (13) erleichtern, wobei die Taschen (25) durch Einfalten der ersten flexiblen Folie (1) oder des Verbundes aus erster (1) und zweiter Folie (4) und/oder durch das Aufbringen weiterer Folienstücke gebildet wird.

14. Gebrauchsfertiges Set zur Unterdruck-Wundbehandlung abdominaler Wunden, umfassend
a) eine Wundauflage nach einem oder mehreren der vorangehenden Ansprüche,
b) mindestens eine flüssigkeitsdurchlässige Schicht (11) zum Aufbringen auf die im Gebrauch von der Wunde abgewandte Seite der Wundauflage, wobei die mindestens eine flüssigkeitsdurchlässige Schicht (11) eine oder mehrere flächenförmige Polster aus einem porösen Polymerschaumstoff, insbesondere aus PU, PVA oder Silikon umfasst,
c) ein luftundurchlässiges Abdeckmaterial (16) zum luftdichten Verschließen der Wunde und der Wundumgebung,
d) ein Unterdruck-Anschlussmittel (18) zur funktionellen Verbindung des Wundraums mit einer außerhalb des Abdeckmaterials befindlichen Unterdruckquelle (21), so dass ein Unterdruck im Wundraum herstellbar ist und Flüssigkeiten aus dem Wundraum absaugbar sind,
wobei alle Komponenten a) bis d) jeweils einzeln steril verpackt vorliegen.

## Claims

1. Wound dressing for use in negative-pressure therapy of abdominal wounds, comprising
- a first flexible film (1) having a first side and a second side, wherein the first side is provided for application to exposed internal organs or to the greater omentum,
- conduits (2) which are applied to the second side of the first film (1) and which are made from a pliable elastomeric material having a thickness (H) of at most 30 mm, wherein the conduits (2) have at least one continuous hollow space (3), wherein the conduits (2) are drainage tubes for medical purposes, wherein the drainage tubes comprise PU, PVC, PE, PET, PTFE, silicone or a mixture thereof, and wherein the conduits (2) and also the first film (1) have a multiplicity of openings, such that a fluid connection can be established between the conduits (2) and the wound space,
and wherein, moreover, the conduits (2) are connectable in a fluid-conducting manner to a negative-pressure source (21) by means of at least one further opening,
and wherein the conduits (2) are shallow and moreover
a) are non-detachably and extensively connected to the second side of the first film (1) with at least 70% of their surface area projected perpendicularly onto the first film (1) or
b) lie on the second side of the first film (1) with at least 85% of their surface area projected perpendicularly onto the first film (1), wherein the conduits (2) are held on the first film (1) by fastening means,
**characterized in that**
at least three conduits (2) are applied to the second side of the first film (1), and **in that** the conduits (2) are applied in a star-shaped arrangement or in a parallel arrangement.

2. Wound dressing according to Claim 1, wherein the conduits (2) are made from a material which has a Shore A hardness of at most 60 (measured as per DIN 53505 of August 2000 at a temperature of 23°C).

3. Wound dressing according to one of the preceding claims, wherein the openings are holes, and wherein the sum of the open surface area of the holes of the first film (1) is at least 0.1% and at most 20% of the areal extent of the film.

4. Wound dressing according to one of the preceding claims, further comprising a second flexible film (4), wherein the second film (4) is applied to the side of the combination of first film (1) and conduits (2) that faces away from the wound during use.

5. Wound dressing according to Claim 4, wherein the second film (4) has a multiplicity of openings distributed across the surface and suitable for fluid to pass through.

6. Wound dressing according to Claim 4, wherein the second film is substantially impermeable to fluid and has only a single opening (10) arranged in the central area.

7. Wound dressing according to one of the preceding claims, wherein the first flexible film (1) and/or, if present, the second flexible film (4) is a thermoplastic film for medical purposes made from PU, PE, PET, PVC, silicone or a mixture thereof.

8. Wound dressing according to one of Claims 4 to 6, wherein the second flexible film (4)
a) is non-detachably connected, at least in places, to the at least three conduits (2),
and/or
b) is non-detachably connected, at least in places, to the first flexible film (1).

9. Wound dressing according to one of the preceding claims, further comprising a conduit plate (8) which is applied to the second side of the first flexible film (1) and on which the ends of the at least three conduits (2) are fastened.

10. Wound dressing according to Claim 9, wherein the conduit plate (8) is in the form of a shallow socket having an inner hollow space (24), and wherein, moreover, the ends of the at least three conduits (2) are introduced such that a fluid connection can be established between the lumina (3) of the conduits (2) and the hollow space of the conduit plate (24).

11. Wound dressing according to one of the preceding claims, furthermore comprising one or more liquid-permeable layers (11) for application to the side of the wound dressing that faces away from the wound during use.

12. Wound dressing according to Claim 11, wherein the liquid-permeable layer (11) comprises a porous polymer foam of PU, PVA or silicone.

13. Wound dressing according to one of the preceding claims, wherein the wound dressing, on the side facing away from the wound during use, has at least one or more peripheral pockets (25) which are open towards the middle of the wound dressing and which have a depth of at most 6 cm, which pockets (25) facilitate the uniform application and laying out of the wound dressing on the wound bed (13), wherein the pockets (25) are formed by folding in the first flexible film (1) or the combination of first film (1) and second film (4) and/or by applying further film pieces.

14. Ready-to-use kit for negative-pressure wound treatment of abdominal wounds, comprising
a) a wound dressing according to one or more of the preceding claims,
b) at least one liquid-permeable layer (11) for application to the side of the wound dressing that faces away from the wound during use, wherein the at least one liquid-permeable layer (11) comprises one or more sheet-like pads made of a porous polymer foam, particularly PU, PVA or silicone,
c) an air-impermeable covering material (16) for air-tight closure of the wound and of the wound environment,
d) a negative-pressure connector (18) for functional connection of the wound space to a negative-pressure source (21) located outside the covering material, such that a negative pressure can be established in the wound space and liquids can be sucked from the wound space,
wherein all of the components a) to d) are each individually packaged in a sterile state.

## Revendications

1. Pansement à utiliser pour la thérapie à pression négative de plaies abdominales, comprenant
- une première feuille flexible (1) avec une première et une deuxième faces, dans laquelle la première face est prévue pour être appliquée sur des organes internes exposés ou sur le grand épiploon,
- des moyens de conduite (2) en un matériau élastomère flexible, installés sur la deuxième face de la première feuille (1), avec une extension en épaisseur (H) de 30 mm au maximum, dans lequel les moyens de conduite (2) présentent au moins une cavité continue (3), dans lequel les moyens de conduite (2) sont constitués par des tuyaux de drainage à usage médical, dans lequel les tuyaux de drainage comprennent PU, PVC, PE, PET, PTFE, silicone ou un mélange de ceux-ci,
et dans lequel aussi bien les moyens de conduite (2) que la première feuille (1) présentent une multiplicité d'ouvertures, de telle manière qu'une communication fluidique puisse être établie entre les moyens de conduite (2) et l'espace de la plaie,
et dans lequel les moyens de conduite (2) peuvent être raccordés en communication fluidique, à travers au moins une autre ouverture, à une source de pression négative (21),
et dans lequel les moyens de conduite (2) sont de forme plate et en outre
a) sont assemblés de façon inséparable et à plat à la deuxième face de la première feuille (1) par au moins 70 % de leur face projetée verticalement sur la première feuille (1)
ou
b) reposent sur la deuxième face de la première feuille (1) par au moins 85 % de leur face projetée verticalement sur la première feuille (1), dans lequel les moyens de conduite (2) sont maintenus sur la première feuille (1) par des moyens de fixation, **caractérisé en ce qu'**au moins trois moyens de conduite (2) sont installés sur la deuxième face de la première feuille (1) et **en ce que** les moyens de conduite (2) sont installés selon un agencement en étoile ou selon un agencement parallèle.

2. Pansement selon la revendication 1, dans lequel les moyens de conduite (2) sont fabriqués en un matériau, qui présente une dureté Shore A (mesurée selon la norme DIN 53505 d'août 2000, à une température de 23°C) de 60 au maximum.

3. Pansement selon l'une quelconque des revendications précédentes, dans lequel les ouvertures sont constituées par des trous et dans lequel la somme de la surface ouverte de trous de la première feuille (1) vaut au moins 0,1 % et au plus 20 % de l'extension superficielle de la feuille.

4. Pansement selon l'une quelconque des revendications précédentes, comprenant en outre une deuxième feuille flexible (4), dans lequel la deuxième feuille (4) est appliquée sur la face du composite formé de la première feuille (1) et des moyens de conduite (2) située, en position d'utilisation, à l'opposé de la plaie.

5. Pansement selon la revendication 4, dans lequel la deuxième feuille (4) présente une multiplicité d'ouvertures réparties sur la surface, qui conviennent pour le passage de fluide.

6. Pansement selon la revendication 4, dans lequel la deuxième feuille est très largement imperméable au fluide et ne présente qu'une seule ouverture (10) disposée dans la région centrale.

7. Pansement selon l'une quelconque des revendications précédentes, dans lequel la première feuille flexible (1) et/ou - si elle est présente - la deuxième feuille flexible (4) est une feuille thermoplastique à usage médical constituée de PU, PE, PET, PVC, silicone ou d'un mélange de ceux-ci.

8. Pansement selon l'une quelconque des revendications précédentes 4 à 6, dans lequel la deuxième feuille flexible (4)
a) est assemblée au moins localement de façon inséparable auxdits au moins trois moyens de conduite (2)
et/ou
b) est assemblée au moins localement de façon inséparable à la première feuille flexible (1).

9. Pansement selon l'une quelconque des revendications précédentes, comprenant en outre une plaque de conduite (8), qui est installée sur la deuxième face de la première feuille flexible (1) et à laquelle les extrémités desdits au moins trois moyens de conduite (2) sont fixées.

10. Pansement selon la revendication 9, dans lequel la plaque de conduite (8) est réalisée sous la forme d'une boîte plate avec une cavité interne (24), et dans lequel les extrémités desdits au moins trois moyens de conduite (2) sont en outre introduites de telle manière qu'une communication fluidique entre les lumina (3) des moyens de conduite (2) et la cavité de la plaque de conduite (24) puisse être établie.

11. Pansement selon l'une quelconque des revendications précédentes, comprenant en outre une ou plusieurs couches (11) perméables au liquide, à appliquer sur la face du pansement située en position d'utilisation à l'opposé de la plaie.

12. Pansement selon la revendication 11, dans lequel la couche perméable au liquide (11) comprend une mousse de polymère poreuse en PU, PVA ou silicone.

13. Pansement selon l'une quelconque des revendications précédentes, dans lequel le pansement présente, sur la face située en position d'utilisation à l'opposé de la plaie, une ou plusieurs poches (25) formées sur le bord et ouvertes vers le centre du pansement, d'une profondeur de 6 cm au maximum, qui facilitent la pose et l'application uniformes du pansement sur le fond de plaie (13), dans lequel les poches (25) sont formées par pliage de la première feuille flexible (1) ou du composite constitué de la première (1) et de la deuxième (4) feuilles et/ou par application d'autres pièces de feuille.

14. Ensemble prêt à l'emploi pour le traitement par des soins à pression négative de plaies abdominales, comprenant
a) un pansement selon une ou plusieurs des revendications précédentes,
b) au moins une couche perméable au liquide (11) à appliquer sur la face du pansement située en position d'utilisation à l'opposé de la plaie, dans lequel ladite au moins une couche perméable au liquide (11) comprend un ou plusieurs tampons plats en une mousse de polymère poreuse, en particulier en PU, PVA ou silicone,
c) un matériau de recouvrement imperméable à l'air (16), pour la fermeture hermétique de la plaie et des alentours de la plaie,
d) un moyen de raccordement de pression négative (18) pour le raccordement fonctionnel de l'espace de la plaie à une source de pression négative (21) se trouvant à l'extérieur du matériau de recouvrement, de telle manière qu'une pression négative puisse être créée dans l'espace de la plaie et que des liquides puissent être aspirés hors de l'espace de la plaie, dans lequel tous les composants a) à d) se trouvent individuellement sous emballage stérile.
